Europäisches Patentamt

European Patent Office

Office européen des brevets

(19)

(11) Veröffentlichungsnummer : **0 082 890**
**B1**

(12) **EUROPÄISCHE PATENTSCHRIFT**

(45) Veröffentlichungstag der Patentschrift :
30.07.86

(21) Anmeldenummer : 81110843.0

(22) Anmeldetag : 30.12.81

(51) Int. Cl.⁴ : **C 12 P   1/02**, G 01 N 33/53,
A 61 K 39/00 // (C12P1/02,
C12R1:72)

(54) Verfahren zur Herstellung von immunbiologischen Präparaten, geeignet für den Nachweis von, die Vorbeugung gegen und/oder die Behandlung von Infektionen durch Candida guilliermondii.

(43) Veröffentlichungstag der Anmeldung :
06.07.83 Patentblatt 83/27

(45) Bekanntmachung des Hinweises auf die Patenterteilung : 30.07.86 Patentblatt 86/31

(84) Benannte Vertragsstaaten :
DE

(56) Entgegenhaltungen :
DE-A- 2 308 037
US-A- 4 138 479
INFECTION AND IMMUNITY, Band 34, Nr. 3, Dezember 1981, Seiten 844-850; R.F. HECTOR et al.: "Immunological relatedness among candida albicans and other pathogenic candida species".
CHEMICAL ABSTRACTS, Band 76, Nr. 13, 1972, Seite 172, Nr. 69834m, Columbus Ohio (USA); T. STRYCHOVA et al.: "Genus candida. XII. Chemical composition of polysaccharides isolated from cell walls of three strains of candida guilliermondii and their immunochemical properties.

(73) Patentinhaber : Sutka, Pál, Dr.
Mészáros u. 60/c
Budapest (HU)

Sutka, Klára, Dr.
Mészáros u. 60/c
Budapest (HU)

(72) Erfinder : Sutka, Pál, Dr.
Mészáros u. 60/c
Budapest (HU)
Erfinder : Sutka, Klára, Dr.
Mészáros u. 60/c
Budapest (HU)

(74) Vertreter : Beszédes, Stephan G. Dr.
Münchener Strasse 80a Postfach 1168
D-8060 Dachau (DE)

EP 0 082 890 B1

**Beschreibung**

Die Erfindung betrifft ein Verfahren zur Herstellung von neuen immunbiologischen Präparaten, die für den Nachweis von, die Vorbeugung gegen und/oder die Behandlung von Infektionen durch Candida guilliermondii bei Menschen und Säugetieren geeignet sind.

Die durch das erfindungsgemäße Verfahren hergestellten immunbiologischen Präparate können besonders vorteilhaft in der Tier- und Humanmedizin zum Nachweis von Infektionen durch Candida guilliermondii, zur Immunisierung der der Infektionsgefahr ausgesetzten Individuen sowie zur Behandlung der betreffenden Krankheit eingesetzt werden.

Bei Wiederkäuern wurde die durch Candida guilliermondii hervorgerufene enzootische Candidiasis (Hauptman, B., Jasinska, S., Siedlicka, B. : Infections of sheep with Saccharomycetes, Med. Vet. *17* [1961], 22) im Jahre 1959 zum ersten Mal festgestellt. Im Laufe späterer Untersuchungen ergab sich, daß Candida guilliermondii bei Rindern in erster Linie die Harn- und Geschlechtsorgane angreift und bei Fehlgeburten beziehungsweise Aborten der Rinder, Entzündungen der Harn- und Geschlechtsorgane und der Geschlechtswege (wie Gebärmutter, Samenblase, Nebenhoden, Euter und Niere) und zahlreichen fortpflanzungsbiologischen Abnormitäten die Rolle des Krankheitserregers spielt. Das Vorkommen der Infektionen durch Candida guilliermondii, ihr klinisches, pathologisches und histologisches Bild, die Ergebnisse von an Pilzen durchgeführten mikrobiologischen Untersuchungen sowie die taxonomische Einteilung der aus infizierten Rinderorganismen isolierten Pilzstämme sind in den folgenden Veröffentlichungen eingehend dargelegt. Sutka P. und Mészáros I. : Tenyészbikák C. guilliermondii fertözöttsége, Magyar Állatorvosok Lapja *33* [1978], 151 ; Sutka P. und Mészáros I. : Candida guilliermondii által fertözött tenyészbikák szerveinek kórbonctani és kórszövettani vizsgálata, Magyar Állatorvosok Lapja *33* [1978], 155 ; Sutka P., Rátz F., Dobolyi Cs. und Novák E. : Tenyészbikák szerveiböl izolált C. guilliermondii törzsek varietas-besorolása és ultrastrukturális vizsgálata, Magyar Állatorvosok Lapja *12* [1978], 837 ; Decun M. und Rosu M. : Investigation of normal and pathological microflora of cervical secretions in cattle, Revta Zootech. Vet. Med. *23* [1973], 39.

Auf Grund der zur Verfügung stehenden Schrifttumsangaben kommt die Infektion durch Candida guilliermondii in erster Linie bei Weiderkäuern vor ; der Pilz ist allerdings auch für den Menschen ein Krankheitserreger. Die Pilze Candida guilliermondii wurden auch in der Humanmedizin bei den unterschiedlichsten Krankheitsbildern nachgewiesen : Bei Infektionen der Harn- und Geschlechtswege (Harding S. A. und Merz W. E. : J. of Clin. Mic. *2* [1975], 222, bei Augeninfektionen (Segal und Mitarbeiter : Mycopath. Mycol. Appl. *54* [1974], 32), bei Entzündungen der Herzinnenhaut (Utley J. R., Milis J. und Mitarbeiter : Circulation *48* [1973], 42) sowie ferner bei systematischer Candidiasis (Kozinn P. J., Taschdjian C. J. und Claire L. : Sab. *7* [1969], 98).

C. guilliermondii ruft bei Menschen und Tieren meist systemische Mykose (Blutvergiftung) hervor, während die am häufigsten diagnostizierten Infektionen durch C. albicans auf den Schleimhäuten des Organismus (Mundhöhle, Vagina und Magen/Darm-Kanal) und auf der Haut und auf den Nägeln Veränderungen verursachen und seltener die Form von allgemeinen Krankheiten annehmen. Deshalb wird bei der Diagnose von C. guilliermondii vor allem systemische Candidiasis nachgewiesen. Für ein frühzeitiges Erkennen der Krankheiten reicht es nicht aus, den Krankheitserreger so schnell wie möglich zu isolieren und ihn morphologisch und biochemisch zu identifizieren, sondern es sind auch moderne und schnelle mykologische immunologische Verfahren erforderlich. Auf dem Gebiet der Humanmedizin wird die Diagnose von systemischer Candidiasis — in erster Linie auf C. albicans bezogen — hauptsächlich mit Verfahren, welche für den Laboratoriumsnachweis von humoralen Abwehrstoffen geeignet sind, nämlich Agglutination, Latexagglutination, indirekte Fluoreszenz, Immundiffusion nach Ouchterloni und Gegenelektrophorese [Counterelektrophorese] durchgeführt, (Taschdjian C. L., P. J. Kozzin, M. B. Cuesta und E. F. Tonu, 1972, Serodiagnosis of Candida infections, Amer. J. Clin. Pathol. *57*, 195 bis 205, [Chemical Abstracts 1978, 4 659 m] ; J. S. Remington, J. D. Gaines und M. A. Gilmer, 1972, Demonstration of Candida precipitins in human sera by counter immunoelectrophoresis, Lancet I, 413 bis 445). In der Hautkunde fanden die Allergene nach Bencard, die zum Auslösen der späten allergischen Immunreaktion geeignet sind, Anwendung. Zur Behandlung der systemischen Candidiasis werden in der Humanmedizin nur verschiedene Pilzmittel beziehungsweise Antimykatika eingesetzt, beispielsweise Amphotericin, B, 5-Fluorcytosin und Mykonasol (Stevens D. A. Drug for systemic fungal infections, Drug Therapy, 1979, *13*, 1 bis 7). Bei Versuchsimmunisierungen wurde bei Kaninchen mit aus toten und abgeschwächten Zellen von C. albicans hergestellten Impfstoffen beziehungsweise Vakzinen gute Immunität erzielt (Balogh E., Kakuk Gy., Halmy K. und Szabolcsi M., Mykosen, 1970, *14*, 385 bis 392). In der Humanmedizin hingegen wurden aus Candida-Arten hergestellte immunbiologische Präparate weder zum Nachweis der späten allergischen Immunreaktion noch für vorbeugende beziehungsweise prophylaktische Impfungen beziehungsweise Heilbehandlungen eingesetzt.

In der Tiermedizin erfolgt die Diagnostizierung von Candidiasis meistens durch pathologischanatomische und histologische Analysen, von Fall zu Fall durch die Isolierung des Krankheiterregers Candida und seine morphologische und biochemische Bestimmung. In diesen Fällen werden auch serologische Untersuchungen mit aus isolierten Pilzstämmen hergestellten Antigenen durchgeführt (Spesiwzewa N. A., 1971 : Metod isledovanija v veterinarnoj mikologii. « Kolos », Moskau). Auf dem Gebiet der Tiermedizin wird der Nachweis von Candidiasis auf immunologischem Wege bis heute noch nicht praktisch angewandt. Für den Tierarzt sind diese Verfahren noch nicht ausreichend ausgearbeitet. Dies bezieht sich besonders auf die allergie-diagnostischen Verfahren. Außerdem wurde bisher nicht über dafür geeignete Antigene und Allergene mit entsprechendem Artenspezificum verfügt.

Für die Heilbehandlung von Candidiasis werden in der Tiermedizin chemotherapeutische Präparate, verschiedene Pilzmittel beziehungsweise Antimykotika, zum Beispiel Nystatin, eingesetzt. Über den immunbiologischen Nachweis von Infektionen durch C. guilliermondii in der Tiermedizin und über für die Vorbeugung gegen sie beziehungsweise Behandlung von ihnen geeignete immunbiologische Präparate sind keine Angaben zu finden.

Bei der Untersuchung der Antigenstruktur der Candida-Arten wurde festgestellt, daß sie aus mehreren Bestandteilen besteht und unter ihnen spezifische und gemeinsame Antigene gefunden werden können (J. Biguet und Mitarbeiter : Electrophorèse et immunochimie de Candida antigenes, Mycopath. Mycol. Appl., 1962, 17, 239).

Mit einem 2-dimensionalen Immunelektrophoreseverfahren wurden 78 Antigenfraktionen aus einer dünnwandigen Zelle von Candida albicans gewonnen. Bei elektronenmikroskopischen Untersuchungen wurde festgestellt, daß die Zellwand aus 5 Schichten besteht (Axelsen N. H. 1973, Quantitative immunoelectrophoretic methods as tools for a polyvalent approach to standardization in the immunochemistry of Candida albicans, Infect. Immun. 7, 949 bis 960 ; Drouchet E. S., Montplaisir 1975, Phénotypes de résistance à la 5-fluorocytosine et relation avec l'ultra-structure chez les Candida. Bull. Soc. Fr. Mycol. Med. 4, 115 bis 118). Bei der serologischen Untersuchung von Candidiasis wurde die Bedeutung der Abwehrstoffe IgM und IgC gegen Candida polysaccharida nachgewiesen. (Müller H. L. 1974 : IgM and IgC-antibodies against Candida polysaccharides in the serodiagnosis of candidiasis, Bull. Soc. Fr. Mycol. Med. 1, 51 bis 52). Es wurde die hohe Antigenwirkung der aus unberührten Zellen und aus den Zellen gewonnenem Mannan-Eiweiß bestehenden Verbindungen bekannt (Tomsikova A., Sikl. D. und Novácko-va D., 1976, Antigenic activity of the cell components of C. albicans, Mykosen, 19, 175 bis 182). Allerdings sind auch gegenwärtig viele Stoffe der Candida-Zelle mit starker Antigenwirkung noch nicht bestimmt. Sowohl für die serologischen Proben als auch für die Allergieproben bedeutet die Herstellung von artspezifischem Antigen zum Nachweis der Candidiasis seit Jahren unverändert ein Problem. Vor allem bei der Herstellung der inneren Zellantigene wurden im Laufe der bisher verwendeten Aufschlußverfahren und Extraktionen (wie Autoklavenbehandlung, Kochen, Autolyse, Naphtholhydrolyse und Phenol- und Acetonbehandlung der Zellen) selbst solche Eiweisse in ihrem natürlichen ursprünglichen Zustand beschädigt, die bei der Spezifität eine Rolle spielen. Gleichzeitig konnte durch diese Verfahren aus den Zellen nur ein bestimmter Teil der inneren antigenartigen Stoffe extrahiert werden, die zwar eine gute Antigenwirkung aufweisen (zum Beispiel Mannan und Mannan-Protein), aber keine die Artspezifität der Hefezelle bestimmenden Eiweisse und keine an die Eiweisse gebundenen verschiedenartigen Haptene enthielten oder solche nur in kleinen Mengen, und so erwiesen sich die so gewonnenen Antigene als nicht vollwertig für die Immundiagnostik, aber hauptsächlich für die Immunprävention und die immunbiologischen Heilbehandlungen (Spesiwzewa N. A. : Metody isledovanija v veterinarnoj mikologii, « Kolos », Moskau. 1971, Chemical Abstracts, 1968, 1 129 988 p und 1976, 175 335 sowie DDR-Patentschrift 113 843).

Zu solchen Antigenen können auch das aus nach der deutschen Patentschrift 23 08 037 beziehungsweise deutschen Offenlegungsschrift 2 308 037 hergestellten Candida-Zellen abgeschiedene Mannan-Hapten und das daran gebundene Heterogen-Eiweiß gezählt werden nach dem Verfahren dieser Druckschrift wird aus der Zellwand von Candida-Hefepilzen, die mindestens einen gemeinsamen Antigenfaktor mit Candida albicans besitzen, reines Mannan durch Kupferkomplexabtrennung isoliert, das so erhaltene Mannan, das nur ein Bestandteil der ganzen Antigenfraktion ist, an ein hefefremdes Trägerprotein gebunden, dann der entstandene Hapten-Träger-Komplex parenteral (durch Injektion) an Warmblüter verabreicht und aus dem Blut der Warmblüter das die Antikörper enthaltende Antiserum isoliert. Dieses Verfahren dient ausschließlich der serologischen Identifizierung der pathogenen Candida-Arten im Laboratorium.

In letzter Zeit wurden durch den Aufschluß von Candida-Zellen auf mechanischem Wege und das verschiedene Zentrifugieren der löslichen Bestandteile Antigene, welche auch gegenwärtig für die Latex-Agglutinations-, Immundiffusions- und Gegen-Immunfällungsverfahren [Counter-Immunpräzipitationsverfahren] verwendet werden, hergestellt (Palmer D. F. und Mitarbeiter, Serodiagnosis of mycotic diseases, Charles C. Thomas, Illinois, USA, 1977 ; US-Patentschrift 4 051 232).

Die für den Nachweis der systemischen Candidiasis-Arten im Laboratorium angewandten bekannten Verfahren erfordern eine anspruchsvollere Vorbereitung als es in der alltäglichen Praxis üblich ist. Bisher wurde nicht über solche artspezifische C. guilliermondii-Antigene, mit denen in der Praxis eines Arztes am Krankenbett oder eines Tierarztes am Lager des erkrankten Tieres oder bei den regelmäßigen Reihenuntersuchungen an Ort und Stelle die Infiziertheit nachgewiesen werden konnte, verfügt.

Bei der Herstellung von Antigenen wurden meistens nur aus Zellen von 48 bis 72 Stunden alten Kulturen der Candida-Arten Stoffe mit Antigenwirkung hergestellt. Bisher beschäftigte man sich sehr wenig mit den bei der verlängerten Zucht in der Candida-Zelle sich abspielenden Veränderungen und den sich dort bildenden Stoffen, obwohl in vivo in infizierten kranken Organismen auch die Produkte von Candida-Zellen verschiedenen Alters gefunden werden können.

Ferner ist aus INFECTION AND IMMUNITY, Band 34, Nr. 3, Dezember 1981, Seiten 844 bis 850 eine vergleichende Untersuchung der antigenen Eigenschaften der durch Aufschluß gewonnenen Auszüge von Blastosporen beziehungsweise deren Membran-Mitochondrien-Fraktion sowie des sich lösenden Cytoplasmateiles und der Zellwand der Blastosporen an unterschiedlichen Candida-Spezies. Bei der Herstellung der Zellmenge werden die durch 1 Monat langes Züchten hergestellten, hauptsächlich subcellulären Fragmente (die Blastosporen sind das subcelluläre Fragment von Hefe, welche von Pseudohyphen umgeben sind, wobei sich aus einer Hyphe oder Pseudohyphe durch Zellvermehrung eine Zelle bildet, welche aber keine vegetative Zelle ist) in einem Kühlschrank bei einer Temperatur von 4 °C auf einem speziellen Nährboden gelagert, wie es insbesondere aus Seite 844, rechte Spalte, vorletzte Zeile bis Seite 845, linke Spalte, Zeile 6 der genannten Druckschrift hervorgeht. Auf dem in der genannten Druckschrift angegebenen modifizierten Sabouraud-Dextrose-Nährboden beginnt also nach der üblichen 48-stündigen vegetativen Zellvermehrung die Entwicklung der subcellulären Komponenten, die Herausbildung von Pseusohyphen und an ihrem Rand von Blastosporen (die in der genannten Druckschrift untersucht wird), zu deren Entwicklung außer dem speziellen Nährboden auch eine längere Zuchtzeit notwendig ist. Hier geht es überhaupt nicht um eine vegetative Zellvermehrung, sondern um aus vegetativen Zellen entstehende extracelluläre Produkte, Blastosporen, Pseudohyphen. Diese Membran-Mitochondrien-Fraktion wird in der Weise weiterverarbeitet, daß sie gegen destilliertes Wasser dialysiert, lyophilisiert und mit 1-Butanol nach der Verfahrenweise von Cohen und Warringa behandelt wird, die Membran-Mitochondrien/Butanol-Mischung unter ständigem Rühren 20 Minten lang auf einem Eisbad gehalten wird und dann noch 10 Minuten vor dem Zentrifugieren stehengelassen wird, worauf die überstehende Flüssigkeit verworfen wird und das Pellet in einem Stickstoffstrom getrocknet, in einem Exsikkator über Nacht gelagert und mit einer mit Phosphat gepufferten Salzlösung 1 Stunde bei 50 °C extrahiert wird sowie aus dem Salzauszug die Proteine mit Ammoniumsulfat gefällt und der Niederschlag erneut in einer nicht-pyrogenen Salzlösung gelöst wird, bevor gegen dasselbe Lösungsmittel dialysiert wird, wonach der Auszug zur gewünschten Konzentration verdünnt und vor dem Gebrauch bei — 20 °C gelagert wird. In der genannten Druckschrift ist auch von der Verwendung der Cytoplasmasubstanzen die Rede. Von der Verarbeitung derselben ist dagegen keine Rede. Auf Seite 845, linke Spalte, Zeilen 10 bis 14 der genannten Druckschrift ist nämlich zwar eine Trennung in Zellwände, Membran-Mitochondrien und lösliche Cytoplasmasubstanzen erwähnt, es werden aber nicht die Zellwände weiterverarbeitet, sondern die Menbran-Mitochondrien-Fraktion.

Weiterhin geht aus CHEMICAL ABSTRACTS, Band 76, Nr. 13, 1972, Seite 172, Nr. 69 834 m nur das Isolieren von Polysacchariden unter anderem aus Stämmen von Candida guilliermondii durch kombinierte alkalische und saure Extraktionen hervor. Nach dieser Druckschrift werden also nur die Polysaccharide, wie Mannan, isoliert. In diesem Fall kommt also im Produkt von den aus dem Fachschrifttum bekannten insgesamt 78 antigenen Fraktionen nur 1 vor.

Außerdem betrifft die US-Patentschrift 4 138 479 die Herstellung des wasserlöslichen Immunpotentierwirkstoffes aus dem Zellwandmaterial von Hefe durch Extrahieren mit einem Gemisch von Wasser und Phenol.

Unter Berücksichtigung der Tatsache, daß die Infektionen durch Candida guilliermondii bei Wiederkäuern (in erster Linie bei Rindern, die vom Gesichtspunkt der Lebensmittelindustrie von grundlegender Bedeutung sind) von ernster Blutvergiftung, fortpflanzungsbiologischen Abnormitäten und solchen der Harn- und Geschlechtsorgane sowie Milchproduktionsstörungen und den damit verbundenen sanitären Problemen begleitete Erkrankungen hervorrufen und infolgedessen sehr große wirtschaftliche Schäden entstehen, sind für den Nachweis der, das Vorbeugen gegen die beziehungsweise die Behandlung der betreffenden Infektionen geeignete tierarzneiliche Präparate, welche besser sind und leichter und umfassender zu handhaben sind als die bekannten, sehr notwendig.

Der Erfindung liegt daher die Aufgabe zugrunde, ein Verfahren zur Herstellung von immunbiologischen Präparaten, geeignet für den Nachweis von, die Vorbeugung gegen und/oder die Behandlung von Infektionen durch Candida guilliermondii, durch welches immunbiologische Präparate, welche auch die artspezifischen Eiweisse der Zelle von Candida guilliermondii sowie andere Stoffe mit Antigenwirkung enthalten und mit welchen in der Praxis eines Arztes am Krankenbett oder eines Tierarztes am Lager des erkrankten Tieres oder bei den regelmäßigen Reihenuntersuchungen an Ort und Stelle die Infiziertheit nachgewiesen werden kann, erhalten werden können, zu schaffen.

Das Obige wurde überraschenderweise durch die Erfindung erreicht.

So wurde erfindungsgemäß überraschenderweise festgestellt, daß bei der verlängerten Zucht in der Candida-Zelle solche neue Stoffe, welche an Ort und Stelle zur Auslösung einer frühe Allergie-Probe und für in einem Rohr durchgeführte Fällungsprobe sowie für die immunbiologische Heilbehandlung von mit dem Pilz infizierten kranken Organismen eingesetzt werden können, entstehen. Solche können überraschenderweise auch mittels bestimmter im folgenden angegebenen Maßnahmen ohne verlängerte Zucht beziehungsweise bei Abtöten der durch 48 bis 72 Stunden langes Vermehren eines Stammes von Candida guilliermondii unter aeroben Bedingungen bei 24 bis 42 °C erhaltenen Kultur hergestellt werden. Die Erfindung beinhaltet ein zum ersten Mal ausgearbeitetes Verfahren zur Herstellung von solchen Stoffen. Mit ihrer Anwendung wird im Veterinärwesen neben den Reihenuntersuchungen des Tierbestandes im Laboratorium auf den Farmen direkt an Ort und Stelle die Durchführung einer schnellen Diagnostizierung möglich, wobei eine auf der allergischen Frühreaktion beruhende Hautprobe und Serum-Rohrausfällung angewandt werden können.

4

Gegenstand der Erfindung ist daher ein Verfahren zur Herstellung von immunbiologischen Präparaten, geeignet für den Nachweis von, die Vorbeugung gegen und/oder die Behandlung von Infektionen durch Candida guilliermondii, durch Züchtung bis zum Erreichen des Endes der Vermehrungsfähigkeit erfolgendes Vermehren eines Stammes von Candida guilliermondii unter aeroben Bedingungen bei 24 bis 42 °C mittels Züchtung auf einem eine durch den Pilz assimilierbare Kohlenstoff- und Stickstoffquelle enthaltenden Nährboden, welches dadurch gekennzeichnet ist, daß die beim Vermehren erhaltene(n) Bevölkerung(en) für die verlängerte Züchtung unter identischen Bedingungen gehalten wird beziehungsweise werden, die Pilzzellen von der Kultur trennt und wäscht oder trocknet sowie in an sich bekannter Weise auf mechanischem Wege (zweckmäßig durch Mahlen oder Ultraschallbehandlung) aufgeschlossen und extrahiert werden, der Auszug mit einem polaren organischen Lösungsmittel behandelt und der erhaltene Niederschlag, gegebenenfalls nach weiterer Reinigung, in an sich bekannter Weise zu einem für immunbiologische Zwecke geeigneten Präparat formuliert wird [Variante a)].

Gegenstand der Erfindung ist auch ein Verfahren zur Herstellung von immunbiologischen Präparaten, geeignet für den Nachweis von, die Vorbeugung gegen und/oder die Behandlung von Infektionen durch Candida guilliermondii, durch 48 bis 72 Stunden langes Vermehren eines Stammes von Candida guilliermondii unter aeroben Bedingungen bei 24 bis 42 °C mittels Züchtung auf einem eine durch den Pilz assimilierbare Kohlenstoff- und Stickstoffquelle enthaltenden Nährboden, welches dadurch gekennzeichnet ist, daß nach gegebenenfalls erfolgendem Weitervermehren der beim Vermehren erhaltene(n) Bevölkerung(en) [Population(en)], vorzugsweise bis zur Herstellung von 2 bis 3 neuen Bevölkerungen [Population(en)], die Pilzzellen von der Kultur getrennt und gewaschen oder getrocknet sowie in an sich bekannter Weise auf mechanischem Wege aufgeschlossen und extrahiert werden sowie gegebenenfalls zum Auszug zur Herstellung eines reineren Wirkstoffes ein polares organisches Lösungsmittel zugegeben wird und der erhaltene Niederschlag, eventuell nach weiterer Reinigung, in an sich bekannter Weise zu einem für immunbiologische Zwecke geeigneten Präparat formuliert wird [Variante b)].

Ferner ist Gegenstand der Erfindung ein Verfahren zur Herstellung von immunbiologischen Präparaten, geeignet für den Nachweis von, die Vorbeugung gegen und/oder die Behandlung von Infektionen durch Candida guilliermondii, durch 48 bis 72 Stunden langes Vermehren eines Stammes von Candida guilliermondii unter aeroben Bedingungen bei 24 bis 42 °C mittels Züchtung auf einem eine durch den Pilz assimilierbare Kohlenstoff- und Stickstoffquelle enthaltenden Nährboden, welches dadurch gekennzeichnet ist, daß die beim Vermehren erhaltene Kultur abgetötet wird, die abgetöteten Zellen abgetrennt werden und sie, gegebenenfalls nach weiterer Reinigung, in an sich bekannter Weise zu einem für immunbiologische Zwecke geeigneten Präparat formuliert werden [Variante c)].

Das erfindungsgemäße Verfahren unterscheidet sich von den obigen bekannten Verfahren grundlegend auch darin, daß es auf die Herstellung von Antigen bezogen außer dem Aufschluß der Zellen von C. guilliermondii auf mechanischem Wege die mit physiologischer Kochsalzlösung durchgeführte Extraktion, die vorteilhaft erfolgende Behandlung des Auszuges mit polaren organischen Lösungsmitteln, vorzugsweise Alkoholen, gegebenenfalls die weitere Reinigung des Niederschlages und das darauffolgende Formen beziehungsweise Umsetzen zu einem immunbiologischen Präparat (zum Beispiel in Form einer antigen-lyophilisierten Ampulle) umfaßt. Die erfindungsgemäß hergestellten löslichen Antigene enthalten infolge der vorteilhafterweise angewandten Extraktion in erster Linie die artspezifischen Eiweisse der Zellen von C. guilliermondii, an die Eiweisse gebundene Haptene sowie andere bisher unbekannte Stoffe mit Antigenwirkung. Daher sind die erfindungsgemäß hergestellten Antigene im Vergleich zu den bisher hergestellten Antigenen, was ihre immunbiologische Wirkung anbelangt, wirksamer.

So stellen beim erfindungsgemäßen Verfahren im Gegensatz zum in der Druckschrift INFECTION AND IMMUNITY, Band 34, Nr. 3, Dezember 1981, Seiten 844 bis 850 beschriebenen Verfahren nicht die Blastosporen (welche bekanntlich entlang der außerhalb der vegetativen Zellen entstehenden Pseudohyphen beziehungsweise deren Produkt bei der auf einem dafür geeigneten Nährboden und unter entsprechenden Bedingungen durchgeführten Züchtung entstehen), sondern die hauptsächlich durch vegetatives Vermehren hergestellten Pilzzellen den Gegenstand der Untersuchung, des Aufschlusses und der Extraktion. Im Gegensatz zum genannten bekannten Verfahren handelt es sich also beim erfindungsgemäßen Verfahren um die Nutzung der maximalen vegetativen Zellvermehrung des Pilzes, da bei diesem der Antigengehalt der vegetativen Zellen und nicht die subcellulär entstehenden Blastosporen untersucht, aufgeschlossen und extrahiert wird. Schon damit allein unterscheidet sich das erfindungsgemäße Verfahren vom Verfahren der genannten Druckschrift grundsätzlich darin, daß verschiedene Materialien verarbeitet werden. Hinzu kommt noch, daß beim erfindungsgemäßen Verfahren im Gegensatz zum Verfahren der genannten Druckschrift, bei welchem die löslichen Antigene der Membran-Mitochondrien und des Cytoplasmas von subcellulär entstehenden Blastosporen ohne die Verarbeitung der Zellwand weiterverarbeitet werden, beim erfindungsgemäßen Verfahren die Pilzzellen oder anders ausgedrückt der Zellenstoff weiterverarbeitet werden, wobei die vegetativen Zellen beziehungsweise ihr ganzen Antigengehalt gefällt wird. In beiden Fällen handelt es sich also um das Weiterverarbeiten von etwas ganz anderem. Dabei unterscheiden sich die Variante a) und teilweise die Variante b) des erfindungsgemäßen Verfahrens von dem der genannten Druckschrift auch darin, daß es sich in der letzteren auf keinen Fall um eine verlängerte Züchtung unter identischen Bedingungen handelt und nicht einmal um eine Weitervermehrung bis zur Herstellung von 2 bis 3 neuen Bevölkerungen, wie es bei der Variante a) des erfindungsgemäßen Verfahrens zwingend und bei der Variante b) des erfindungsgemäßen Verfahrens fakultativ der Fall ist.

Aber auch in den Verfahrensmaßnahmen selbst, welche wie bereits dargelegt ohnehin an einem grundlegend verschiedenen Material durchgeführt werden, unterscheidet sich das erfindungsgemäße Verfahren von dem der Druckschrift INFECTION AND IMMUNITY, Band 34, Nr. 3, Dezember 1981, Seiten 844 bis 850 grundlegend. So ist erfindungsgemäß im Gegensatz zur genannten Druckschrift, nach welcher die gewaschenen Blastosporen in einem tris-(Hydroxymethyl)-amino-methan-Puffer gewaschen werden, ein solches überhaupt nicht vorgesehen. Auch wird im erfindungsgemäßen Verfahren im Gegensatz zum Verfahren der genannten Druckschrift, nach welchem die Membran-Mitochondrien-Fraktion gegen destilliertes Wasser dialysiert wird, eine Dialyse überhaupt nicht durchgeführt. Ferner wird beim erfindungsgemäßen Verfahren im Gegensatz zum Verfahren der genannten Druckschrift, bei welchem im Anschluß an das Lyophilisieren eine Behandlung mit 1-Butanol erfolgt, zunächst vor dem Versetzen mit dem polaren organischen Lösungsmittel extrahiert, wozu vorzugsweise eine physiologische Kochsalzlösung verwendet wird, und erst der so erhaltene Auszug mit dem polaren organischen Lösungsmittel versetzt. Selbst wenn in beiden Fällen gleiche Materialien verarbeitet würden, was, wie es oben nachgewiesen wurde, eindeutig nicht der Fall ist, wäre dies ein wesentlicher Unterschied für die Eigenschaften der erhaltenen Produkte, da schon allein durch das Fehlen der Extraktion beim Verfahren der genannten Druckschrift etwas ganz anderes mit dem polaren organischen Lösungsmittel versetzt wird als beim erfindungsgemäßen Verfahren. Zwar wird auch beim Verfahren der genannten Druckschrift gemäß Seite 845, linke Spalte, Zeile 24 bis 25 extrahiert, und zwar mit einer phosphatgepufferten Salzlösung, dieser Extraktion wird aber erst der getrocknete Niederschlag der Fällung mit 1-Butanol unterworfen, was, wie aus dem Obigen hervorgeht, ein wesentlicher Unterschied ist. Auch dies ein Grund dafür, daß in den Produkten des erfindungsgemäßen Verfahrens alle in vegetativen Pilzzellen vorkommenden mit Alkohol ausfällbaren Polysaccharide, Eiweiße, Lipoproteine und sonstige Antigene bisher unbekannter Zusammensetzung enthalten sind. Auch das bei den Variaten a) und b) des erfindungsgemäßen Verfahrens nach dem Waschen und Trocknen der Pilzzellen durchgeführte Aufschließen auf mechanischem Wege hat mit dem Aufschließen der im tris-(Hydroxymethyl)-aminomethan-Puffer suspendierten Blastosporen durch ballistische Wirkung in einem Homogenisierapparat nach dem Verfahren der genannten Druckschrift nichts zu tun, da es sich im erstgenannten Fall um das Aufschließen von Pilzzellen, im letztgenannten Fall dagegen um das Aufschließen von Blastosporen handelt, so daß die Verhältnisse in beiden Fällen völlig verschieden sind. Auch auf die Variante c) des erfindungsgemäßen Verfahrens hat die genannte Druckschrift keinen Bezug, da in der genannten Druckschrift kein Abtöten einer beim Vermehren erhaltenen Kultur vorgesehen ist, wobei von einer Formaldehydbehandlung keine Rede ist.

Zusammenfassend ergibt sich, daß auf Grund der oben dargelegten grundlegenden Unterschiede zwischen dem erfindungsgemäßen Verfahren und dem Verfahren der genannten Druckschrift im verarbeiteten Material (vegetative Pilzzellen gegenüber subzelluläre entstehende Blastosporen und der ganze Antigengehalt der vegetativen Pilzzelle gegenüber einzelnen Blastosporenfraktionen) und in den angewandten Verfahrensmaßnahmen das erfindungsgemäße Verfahren vom Verfahren der genannten Druckschrift grundlegend verschieden ist.

Analoges ergibt sich gegenüber der Druckschrift CHEMICAL ABSTRACTS, Band 76, Nr. 13, 1972, Seite 172, Nr. 69834m. Im Gegensatz zum Verfahren dieser Druckschrift handelt es sich erfindungsgemäß nicht lediglich um das Isolieren der Polysaccharide, wie von Mannan, sondern um die Herstellung eines Produktes, welches neben diesen auch die artspezifischen Eiweiße der Zelle von Candida guilliermondii sowie andere Stoffe mit Antigenwirkung enthält. So sind in den nach dem erfindungsgemäßen Verfahren erhaltenen Produkten alle in vegetativen Pilzzellen vorkommenden mit Alkohol ausfällbaren Polysaccharide, Eiweiße, Lipoproteine und sonstige Antigene bisher unbekannter Zusammensetzung enthalten, während in den nach dem Verfahren der genannten Druckschrift erhaltenen Produkten nur 1 der aus dem Fachschrifttum bekannten insgesamt 78 antigenen Fraktionen vorliegt.

Die beim erfindungsgemäßen Verfahren gegenüber dem Verfahren der Druckschrift INFECTION AND IMMUNITY, Band 34, Nr. 3, Dezember 1981, Seiten 844 bis 850 bestehenden oben dargelegten grundsätzlichen Unterschiede bestehen auch gegenüber dem Verfahren der deutschen Patentschrift 23 08 037 beziehungsweise deutschen Offenlegungsschrift 2 308 037. So unterscheiden sich die erfindungsgemäß hergestellten aufgeschlossenen Antigene auch vom nach der deutschen Patentschrift 23 08 037 beziehungsweise deutschen Offenlegungsschrift 2 308 037 hergestellten C. guilliermondii-Antigen im wesentlichen darin, daß sie neben Mannan, Lipoiden und anderen Haptenen die artspezifischen Eiweiße der Zelle von C. guilliermondii sowie andere Stoffe mit Antigenwirkung enthalten. Erfindungsgemäß wird also aus den Zellen von Candida guilliermondii nicht nur das Mannan isoliert, sondern die ganze Antigenfraktion, welche die artspezifischen Eiweiße der Zellen von Candida guilliermondii, die an die Eiweiße gebundenen Haptene sowie andere Stoffe bisher unbekannter antigener Wirkung enthalten. Hierzu trägt auch bei, daß beim erfindungsgemäßen Verfahren bei den Varianten a) und b) im Gegensatz zum Verfahren der genannten Druckschriften ein Waschen oder Trocknen und Aufschließen durchgeführt werden, wobei insbesondere das beim Verfahren der genannten Druckschriften fehlende Aufschließen dafür verantwortlich ist, daß beim erfindungsgemäßen Verfahren im Gegensatz zum Verfahren der genannten Druckschriften die artspezifischen Eiweiße der Zelle von C. guilliermondii sowie andere Stoffe mit Antigenwirkung in das Produkt gelangen. Auch die Tatsache, daß beim Verfahren der genannten Druckschriften das Mannan, vorzugsweise durch Umsetzen mit polychlorierten Triazinen oder mit Bromacetylbromid, an ein hefefremdes Trägerprotein gebunden wird, um einen Hapten-Trägerkomplex herzustellen, zeigt, daß beim Verfahren der genannten Druckschrift keine artspezifischen Eiweiße in das Produkt gelangen, denn sonst wäre es nicht erforderlich, hefefremde Trägerproteine einzubauen. Im Gegensatz dazu ist beim erfindungsgemäßen Verfahren so etwas nicht vorgesehen, da es wie bereits dargelegt auf die Isolierung der ganzen Antigenfraktion abgestellt ist. Auch das Verwenden einer abgetöteten Kultur gemäß der Variante c) des erfindungsgemäßen Verfahrens ist in den genannten Druckschriften nicht vorgesehen. Ein weiterer Unterschied besteht darin, daß, während die beschriebenen immunbiologischen Präparate ausschließlich zur Identifikation der infizierenden Candida-Arten im Laboratorium geeignet sind, die erfindungsgemäß hergestellten Präparate eben infolge der Gegenwart von artspezifischen Bestandteilen gleichermaßen zum Nachweis und vorbeugenden Behandlung von frühen und späten Infiziertheitsstadien angewandt werden kann.

Auch vom Verfahren der US-Patentschrift 4 138 479 unterscheidet sich das erfindungsgemäße Verfahren grundlegend genauso wie von den oben genannten anderen Druckschriften. So wird nach dem erfindungsgemäßen Verfahren im Gegensatz zum Verfahren der genannten Druckschrift wiederum die ganze Antigenfraktion, welche in den Zellen von Candida guilliermondii zu finden ist, und zu welcher nicht nur die antigenen Stoffe in der Zellwand, sondern auch die artspezifischen Eiweiße der Zelle, die an die Eiweiße gebundenen Haptene sowie bisher nicht bekannte andere Stoffe antigener Wirkung gehören, isoliert, wobei noch dazu in der genannten Druckschrift nicht einmal Candida guilliermondii erwähnt ist. Gleichzeitig erleiden beim in der genannten Druckschrift beschriebenen warmen Extrationsverfahren mit Phenol die einzelnen Bestandteile der antigenen Fraktion nach dem Aufschluß eine qualitative Umwandlung, was die Lösung der erfindungsgemäß gestellten Aufgabe vereiteln würde. Es ergibt sich insgesamt, daß von den Maßnahmen des erfindungsgemäßen Verfahrens in der genannten Druckschrift lediglich die Maßnahme des Extrahierens schlechthin enthalten ist.

Auch werden durch das erfindungsgemäße Verfahren im Gegensatz zu den bekannten Verfahren solche artspezifische C. guilliermondii-Antigene, mit denen in der Praxis eines Arztes am Krankenbett oder eines Tierarztes am Lager des erkrankten Tieres oder bei den regelmäßigen Reihenuntersuchungen an Ort und Stelle die Infiziertheit nachgewiesen werden kann, erhalten.

Durch das erfindungsgemäße Verfahren kann also das Ausmaß der Infiziertheit mit Candida guilliermondii nachgewiesen werden und es können regelmäßige vorbeugende Reihenuntersuchungen durchgeführt und die Immunisierung gelöst werden.

Nur das fortpflanzungsbiologische Ausfiltern von infizierten Individuen in den Beständen sichert den landwirtschaftlichen Betrieben Kosteneinsparungen bei der Tierhaltung und -fütterung sowie sonstige Kosteneinsparungen. Da auch Menschen infiziert werden können, haben die diagnostischen Reihenuntersuchungen auch Bedeutung für das Gesundheitswesen und die Lebensmittelhygiene. Die Anwendung der Erfindung ist besonders in fortpflanzungsbiologischer Hinsicht bei Rindern von wirtschaftlichem Interesse. Alle diese Stoffe, die zur Diagnose und Immunisierung geeignet sind, sind auch bei Menschen wirksam. Daher sind die erfindungsgemäß hergestellten Präparate in entsprechender Form — im Hinblick auf die Rolle des Pilzes als Krankheitserreger bei Menschen — auch in der Humanmedizin anwendbar.

Bei der Variante a) des erfindungsgemäßen Verfahrens werden also für die verlängerte Züchtung zu weiterer Vermehrung unfähige Mikroorganismen verwendet.

Bei der Variante a) des erfindungsgemäßen Verfahrens beträgt die Dauer des Vermehrens meistens 48 bis 72 Stunden, sie kann aber im Gegensatz zu den Varianten b) und c) auch außerhalb dieses Bereiches liegen.

Vorzugsweise wird beziehungsweise werden bei der Behandlung des Auszuges als polares organisches Lösungsmittel 1 oder mehr Alkanol(e) mit 1 bis 4 Kohlenstoffatom(en), insbesondere Äthanol, verwendet.

Zweckmäßig wird das Formen beziehungsweise Umsetzen zum für immunbiologische Zwecke geeigneten Präparat zu einer Form, welche in der Human- oder Tiermedizin anwendbar ist, durchgeführt.

Vorzugswiese wird im Falle der Varianten a) und b) des erfindungsgemäßen Verfahrens das Formen zum für immunbiologische Zwecke geeigneten Präparat zu einem Allergen, löslichen Antigen oder Impfstoff durchgeführt.

Es ist bevorzugt, im Falle der Variante c) des erfindungsgemäßen Verfahrens das Formen zum für immunbiologische Zwecke geeigneten Präparat zu einem korpuskulären Antigen durchzuführen.

Vorteilhaft werden beim Vermehren beziehungsweise Weitervermehren Temperaturen von 32 bis 38 °C angewandt. Zweckmäßig wird dabei im Falle der Varianten a) und b) des erfindungsgemäßen Verfahrens ein flüssiger Nährboden und im Falle der Variante c) des erfindungsgemäßen Verfahrens ein flüssiger oder fester Nährboden verwendet. Auch isrt insbesondere bei den Varianten b) und c) des erfindungsgemäßen Verfahrens eine Zeitdauer von 48 bis 72 Stunden beim Vermehren beziehungsweise ersten Vermehren bevorzugt.

Vorzugsweise wird zum Waschen und/oder Extrahieren eine physiologische Kochsalzlösung verwendet.

Nach einer vorteilhaften speziellen Ausführungsform des erfindungsgemäßen Verfahrens zur Herstellung von Serum mit hohem Präzipitingehalt, geeignet als Reagens für den Laboratoriumsnachweis von Infektionen durch Candida guilliermondii, werden Kaninchen mit nach der Verfahrensvariante a) erfindungsgemäß hergestellten immunbiologischen Präparaten auf übliche Weise immunisiert, dann die Tiere entblutet und das Serum der Tiere auf übliche Weise isoliert.

Nach einer anderen vorteilhaften speziellen Ausführungsform des erfindungsgemäßen Verfahrens zur Herstellung von Serum mit hohem Agglutiningehalt, geeignet als Reagens für den Laboratoriumsnachweis von Infektionen durch Candida guilliermondii, werden Kaninchen mit nach der Verfahrensvariante b) oder c) erfindungsgemäß hergestellten immunbiologischen Präparaten auf übliche Weise immunisiert, dann die Tiere entblutet und das Serum der Tiere auf übliche Weise isoliert.

Gegebenenfalls kann auch der nach der Variante b) erhaltene Auszug der Extraktion nach der Einstellung des gewünschten Eiweißgehaltes als Antigen bei Laboratoriumsuntersuchungen verwendet werden.

Zum Reinigen des nach den Varianten a) beziehungsweise b) des erfindungsgemäßen Verfahrens erhaltenen Niederschlages können bekannte Verfahren der Eiweißreinigung, zum Beispiel Chromatographieren, Ultrafiltrieren, Dialyse, Anwendung von Filterhilfsstoffen beziehungsweise Detergentien oder Elektrophorese, angewandt werden.

Das Aufschließen der Zellen auf mechanischen Wege kann durch Mahlen und/oder Ultraschallbehandlung und/oder mit einem X-Press-Gerät (ein Aufschließgerät für Laboratoriumszwecke dessen Wirkungsprinzip wie folgt ist : Die aufzuschließende Substanz wird in den Metallbehälter des Gerätes eingebracht und in diesem mit Trockeneis eingefroren. Unter entsprechendem Druck wird das gefrorene Material dann durch eine sehr kleine Öffnung hindurch in einen anderen Behälter gepreßt. Durch die dabei auf die Substanz einwirkende mechanische Kraft erfolgt der Aufschluß) bewerkstelltigt werden.

Im folgenden werden nach der Variante a) des erfindungsgemäßen Verfahrens hergestellte immunbiologische Präparate mit Cg1, nach der Variante b) des erfindungsgemäßen Verfahrens hergestellte immunbiologische Präparate mit Cg2 und nach der Variante c) des erfindungsgemäßen Verfahrens hergestellte immunbiologische Präparate mit Cg3 bezeichnet.

Die immunbiologischen Präparate Cg1 sind Zellauszüge, die aus einer verlängerten Züchtung unterworfenen Zellen von Candida guilliermondii abgetrennt wurden und auch Eiweiß enthalten. Die wichtigsten Bestandteile dieser Zellauszüge sind die Toxine von Candida guilliermondii. Die immunbiologischen Präparate Cg1 sind in erster Linie geeignet, die Infektionen durch Candida guilliermondii durch das Auslösen einer immubiologischen allergischen Frühreaktion mit einer intrakutanen Probe nachzuweisen. Gleichzeitig sind sie geeignet, mit einer Fällungsprobe (Präzipitationsprobe), bei der ein Durham- oder Kapillarröhrchen verwendet wird, die zirkulierenden Abwehrstoffe nachzuweisen. Außerdem verursachen diese Präparate, wenn sie in den lebenden Organismus gelangen, die Erzeugung von Abwehrstoffen, die geeignet sind, die schon entstandene Infektion durch Candida guilliermondii abzuschaffen.

Die immunbiologischen Präparate Cg2, die aus Zellen von Candida guilliermondii, welche einer kurz verlängerten Züchtung unterworfen wurden, abgetrennt wurden, enthalten wesentlich mehr Eiweiß als die immunbiologischen Präparaten Cg1 und gleichzeitig ist ihr Toxingehalt niedrig. Diese immunbiologischen Präparate sind vor allem dazu geeignet, der Infektionsgefahr ausgesetzte lebende Organismen zu immunisieren. Außerdem können sie als Diagnosemittel zum Nachweis der immunbiologischen allergischen Spätreaktion mit einer intrakutanen Probe sowie ferner als Antigene zum Nachweis der Infektion für Latexagglutinations-, Immundiffusions- und Gegenimmun-Elektrophoreseverfahren [Counterimmun-Elektrophoreseverfahren] eingesetzt werden.

Die immunbiologischen Präparate Cg3 bestehen aus abgetöteten ganzen Zellen von Candida guilliermondii und werden in erster Linie zum Nachweis des frühen Stadiums der Infektionen durch Candida guilliermondii in Laboratoriumsverfahren, zum Beispiel bei Agglutinationsuntersuchungen und indirekten Fluoreszenzuntersuchungen, oder zur Herstellung der für die ABR/ Abortus-Bang-Ringprobe notwendigen gefärbten Antigene (siehe Hutyra F., I. Marek, R. Manninger und I. Mocsy Spezielle Pathologie und Therapie der Haustiere, VEB Gustav-Fischer-Verlag, Jena, 1959) verwendet.

Zur Herstellung dieser 3 immunbiologischen Präparate können als erzeugende Mikroorganismen vorteilhaft und mit Erfolg alle aus dem Organismus der infizierten Individuen abgesonderten Stämme von Candida guilliermondii verwendet werden. Zu diesem Zweck können allerdings auch die bekannten internationalen Standardstämme von Candida guilliermondii verwendet werden, zum Beispiel der im Stammkatalog LIST OF CULTURES 1968 der Stammsammlung CENTRALBUREAU VOOR SCHIMMELCULTURES, Delft, Niederlande, genannte Stamm Nr. CBS-566 und die ebenfalls im Stammkatalog LIST OF CULTURES 1968 der Stammsammlung CENTRALBUREAU VOOR SCHIMMELCULTURES, Delft, Niederlande, und zwar auf Seite 210, Zeilen 24 von unten bis 14 von unten, aufgeführten Stämme von Candida guilliermondii

Candida guilliermondii (Cast.) Lang & Guerra 463, Hungarian Ampelological Inst. ; 566, T, Castellani ; 1909 (T, C. melibiosi), fr. flower of Gentiana imbricata, Grüss ; 1951, fr. washed beer bottle, Een ; 2021 = ATCC 9058, Burkholder ; 2022 (T, Torula fermentati), fr. air, Saito ; 2023, fr. blood, Arzt via Ota ; 2024, fr. equine ulcer, Lindner via Ota ; 2025, fr. butter milk, Lab. Microbiol. Delft ; 2033 (T, T. xylinus), Sato ; 2077, fr. lung, CBS ; 2262, fr. leather ; 2672, fr. cystitis, Ota ; 2682, fr. bull's semen, Austwick ; 2821, fr. sputum, Sanatorium Berg en Bosch, Bilthoven ; 2891, fr. leather ; 2930, Friedrich ; 4236, fr. child's kidney, Friedrich ; 5059, fr. Cossidae larva, van der Walt ; 5241, fr. sake starter, Kodama ; 5265 (T, Tr. appendiculare), fr. appendix, Batista ; 5289, fr. sake starter, Kodama ; 5483, fr. culture contaminant, Charles

sowie die im Stammkatalog der Stammsammlung AMERICAN TYPE CULTURE COLLECTION, Catalogue of Strains I, Fourteenth Edition, 1980 auf Seite 261, linke Spalte, Zeile 32 von unten bis rechts Spalte, Zeile 6 aufgeführten Stämme von Candida guilliermondii

Candida guilliermondii (Castellani) Langeron et Guerra

6260 A. Castellani (Monilia guilliermondii). (ATCC 7350, CBS 566, NRRL Y-324) Bronchomycosis. Type culture (Lodder, J. and N.J.W. Kreger-van Rij. « The Yeasts — A Taxonomic Study ». 1952, p. 523. North Holland Pub. Co., Amsterdam). (Medium 200 26 C)

9058 P. R. Burkholder. (CBS 2021 ; IFO 0566, NRRL Y-488) Production of : riboflavin (Science 101 (2616) : 180-181, 1945 ; U.S. Pat. 2,363,277) and citric acid (U.S. Pat. 3,669,839). (Medium 200 24 C)

9390 A. E. de Area Leao 2029 ● J. E. Mackinnon 642. J. Bact. 49 : 317-334 (1945). Medium 200 24 C)

14242 A. Castellani. Medium 200 26 C)

20118 Takeda Chem. Ind., Ltd. ● IFO 0838 ● Y. Ohara 57. Patent strain. (Medium 200 24 C)

20316 Morinaga Milk Ind. Co., Ltd. M-7006 ● Y. Harada. Degradation of hydrocarbon, producing microbial proteinous substance (U.S. Pat. 3,855,063). (Medium 200 24 C)

20318 Morinaga Milk Ind. Co., Ltd. M-7010 ● Y. Harada. Degradation of hydrocarbon, producing microbial proteinous substance (U.S. Pat. 3,855,063). (Medium 200 24 C)

20403 Takeda Chem. Ind., Ltd IFO 1651. Production of yeast biomass (U.S. Pat. 3,909,352). (Medium 200 24 C)

20474 Pfizer Inc. 525 (FD 22547). Food additive petition culture. (Medium 200 28 C)

22948 H. F. Hasenclever B3344#2 Columba livia (Medium 200 24 C)

22949 H. F. Hasenclever B3341#1 Columba livia (Medium 200 24 C)

26860 T. Stryckova CCY-29-4-6 ● RIBM ● CHTU. Cell wall poiysaccharides (Folia Microbiol. 16 : 417-425, 1971). (Medium 200 24 C)

28873 CBS 5265 ● A. C. Batista. Human appendix. Type culture of Trichosporon appendiculare (Rev. Assoc. Med Brasil 5 351, 1959). (Medium 200 24 C)

32542 H. S. Randhawa VCM/F/4. Psidium guajava, guava fr (Medium 200 24 C)

34134 G. D. Roberts ST-105. Clinical specimen. Identification (J. C Microbiol. 3 : 302-325, 1976). (Medium 200 24 C)

38290 V. Hopsu-Havu Tu 62304. Human leg. (Medium 200 24 C)

und ferner die in Magyar Állatorvosok Lapja 1978, Nr. 12, Seiten 837 bis 843 abgehandelten Stämme mit den in den folgenden Tabellen 1 und 2 zusammengestellten Ergebnissen der Gärungsuntersuchung beziehungsweise Assimilationsuntersuchung der Kohlenstoffquellenverwertung.

(Siehe Tabellen Seite 10 ff.)

Tabelle 1

Gärungsuntersuchung der Kohlenstoffquellenverwertung von Stämmen von Candida guilliermondii

| Lau-fende Nr. | Nr. des isolierten Stammes | Organ, aus welchem die Isolierung er-folgte | G | L | Su | Ma | Ga | R | Tr | Ce | Me | I |
|---|---|---|---|---|---|---|---|---|---|---|---|---|
| 1. | 1387 | Hode | + | - | + | - | K | + | K | - | - | + |
| 2. | 1760 | Nebenhode | + | - | + | - | K | K | K | - | - | + |
| 3. | 1765 | Nebenhode | + | - | + | - | K | K | K | - | - | + |
| 4. | 1811 | Niere | + | - | + | - | K | K | K | - | - | + |
| 5. | 1814 | Niere | + | - | + | - | K | + | K | - | - | + |
| 6. | 1917 | Hode | + | - | + | - | K | K | K | - | - | + |
| 7. | 1918 | Samenblase | + | - | + | - | K | K | K | - | - | + |
| 8. | 1922 | Nebenhode | + | - | + | - | K | K | K | - | - | + |
| 9. | 1953 | Nebenhode | + | - | + | - | K | K | K | - | - | + |

0 082 890

Tabelle 1 (Fortsetzung)

| Laufende Nr. | Nr. des isolierten Stammes | Organ, aus welchem die Isolierung erfolgte | G | L | Su | Ma | Ga | R | Tr | Ce | Me | I |
|---|---|---|---|---|---|---|---|---|---|---|---|---|
| 10. | 1956 | Samenblase | + | – | + | – | K | + | K | – | – | + |
| 11. | 1973 | Milz | + | – | + | – | K | K | K | – | – | + |
| 12. | 2317 | Bauchspeicheldrüse | + | – | + | – | K | K | K | – | – | + |
| 13. | 2520 | Niere | + | – | + | – | K | K | K | – | – | + |
| 14. | 2539 | Nebenhode | + | – | + | – | K | K | K | – | – | + |
| 15. | 2718 | Samenblase | + | – | + | – | K | K | K | – | – | + |
| 16. | 2742 | Lendenlymphknoten | + | – | + | – | K | K | K | – | – | + |
| 17. | 2744 | Nebenhode | + | – | + | – | K | K | K | – | – | + |
| 18. | 2750 | Samenblase | + | – | + | – | K | K | K | – | – | + |

0 082 890

## Tabelle 1 (Fortsetzung)

| Laufende Nr. | Nr. des isolierten Stammes | Organ, aus welchem die Isolierung erfolgte | G | L | Su | Ma | Ga | R | Tr | Ce | Me | I |
|---|---|---|---|---|---|---|---|---|---|---|---|---|
| 19. | 2831 | Samenblase | + | – | + | – | K | K | K | – | – | + |
| 20. | 2904 | Nebenhode | + | – | + | – | K | K | K | – | – | + |
| 21. | 2998 | Nebenhode | + | – | + | – | K | K | K | – | – | + |
| 22. | 3018 | Samenblase | + | – | + | – | K | K | K | – | – | + |
| 23. | 3891 | Nebenhode | + | – | + | – | K | + | K | – | – | + |
| 24. | 4922 | Nebenhode | + | – | + | – | K | + | K | – | – | + |
| 25. | 5434 | Niere | + | – | + | – | K | K | K | – | – | + |

Zeichenerklärung (auch für die Tabelle 2)

+ = starke Gärung

K = schwache Gärung

G = Glucose, Ga = Galaktose, Sor = 1-Sorbose, Su = Rohrzucker, Ma = Maltose, Ce = Cellobiose, Tr = Trehalose, L = Milchzucker, Me = Melibiose, R = Raffinose, Mz = Melecitose, I = Inulin, Ke = Stärke, X = d-Xylose, I-A = l-Arabinose, d-A = d-Arabinose, Ri = d-Ribose, Rh = l-Rhamnose, Et = Äthylalkohol, Gly = Glyzerin, Er = Erythrit, Rib = Ribit, Gal = Galaktit, Mi = d-Mannit, Glu = d-Glucit, MDG = α-Methyl-D-glucosid, S = Salicin, T = dl-Milchsäure, B = Bernsteinsäure, C = Zitronensäure und In = Inosit

## Tabelle 2

Assimilationsuntersuchung der Kohlenstoffquellenverwertung von Stämmen von Candida guilliermondii

| Laufende Nr. | Nr. des isolierten Stammes | G | Ga | Sor | Su | Mn | Ce | Tr | L | Me | R | Mz | I | Ke | X | 1-A | d-A | Ri | Rh | Et | Gly | Er | Rib | Gal | Mi | Glu | MXG | S | T | B | C | In |
|---|---|---|---|---|---|---|---|---|---|---|---|---|---|---|---|---|---|---|---|---|---|---|---|---|---|---|---|---|---|---|---|---|
| 1. | 1487 | + | + | + | + | + | + | + | − | + | + | + | + | − | + | + | + | − | − | + | + | − | + | + | + | + | + | + | + | + | + | − |
| 2. | 1750 | + | + | + | + | + | + | + | − | + | + | + | + | − | + | + | + | − | − | − | + | − | + | + | + | + | + | + | + | + | + | − |
| 3. | 1765 | + | + | + | + | + | + | + | − | + | + | + | + | − | + | + | + | − | − | − | + | − | + | + | + | + | + | + | − | + | + | − |
| 4. | 1811 | + | + | + | + | + | + | + | − | + | + | + | + | − | + | + | + | − | − | + | + | − | + | + | + | + | + | + | − | + | + | − |
| 5. | 1814 | + | + | + | + | + | + | + | − | + | + | + | + | − | + | + | + | − | − | + | − | + | + | + | + | + | + | − | + | + | − |
| 6. | 1817 | + | + | + | + | + | + | + | − | + | + | + | + | − | + | + | + | − | + | + | − | + | + | + | + | + | + | + | + | + | − |
| 7. | 1818 | + | + | + | + | + | + | + | − | + | + | + | + | − | + | + | + | − | − | − | + | + | + | + | + | + | + | + | − | + | + | − |
| 8. | 1822 | + | + | + | + | + | + | + | − | + | + | + | + | − | + | + | + | − | − | + | − | + | + | + | + | + | + | − | + | + | − |
| 9. | 1853 | + | + | + | + | + | + | + | − | + | + | + | + | − | + | + | + | − | − | + | − | + | + | + | + | + | − | + | + | − |
| 10. | 1858 | + | + | + | + | + | + | + | − | + | + | + | + | − | + | + | + | − | − | + | − | + | + | + | + | + | − | + | + | − |
| 11. | 1873 | + | + | + | + | + | + | + | − | + | + | + | + | − | + | + | − | + | + | − | + | + | + | + | + | + | − | + | + | − |
| 12. | 2317 | + | + | + | + | + | + | + | − | + | + | + | + | − | + | + | − | − | + | − | + | + | + | + | + | + | − | + | + | − |
| 13. | 2520 | + | + | + | + | + | + | + | − | + | + | + | + | − | + | + | − | − | + | − | + | + | + | + | + | − | + | − |
| 14. | 2533 | + | + | + | + | + | + | + | − | + | + | + | + | − | + | + | − | − | + | − | + | + | + | + | + | − | + | − |
| 15. | 2718 | + | + | + | + | + | + | + | − | + | + | + | + | − | + | + | − | − | + | − | + | + | + | + | − | + | − |
| 16. | 2742 | + | + | + | + | + | + | + | − | + | + | + | + | − | + | + | − | − | + | − | + | + | + | + | − | + | − |
| 17. | 2744 | + | + | + | + | + | + | + | − | + | + | + | + | − | + | + | − | − | + | + | − | + | + | + | + | − | + | − |
| 18. | 2750 | + | + | + | + | + | + | + | − | + | + | + | + | − | + | + | − | − | + | − | + | + | + | + | − | + | − |
| 19. | 2831 | + | + | + | + | + | + | + | − | + | + | + | + | − | + | + | − | − | − | + | − | + | + | + | + | + | − | + | − |
| 20. | 2304 | + | + | + | + | + | + | + | − | + | + | + | + | − | + | + | − | − | − | + | − | + | + | + | + | − | + | − |
| 21. | 2372 | + | + | + | + | + | + | + | − | + | + | + | + | − | + | + | − | − | − | + | − | + | + | + | + | − | + | − |
| 22. | 3018 | + | + | + | + | + | + | + | − | + | + | + | + | − | + | + | − | − | + | − | + | + | + | + | − | + | − |
| 23. | 3891 | + | + | + | + | + | + | + | − | + | + | + | + | − | + | + | − | − | − | + | − | + | + | + | + | − | + | − |
| 24. | 4922 | + | + | + | + | + | + | + | − | + | + | + | + | − | + | + | − | − | − | + | − | + | + | + | + | − | + | − |
| 25. | 5443 | + | + | + | + | + | + | + | − | + | + | + | + | − | + | + | − | − | + | − | + | + | + | + | + | − | + | − |

Für die Züchtung des Stammes von Candida guilliermondii wird zweckmäßig von einer Vorkultur (einem bei der Hauptgärung vorgezüchteten Stamm) welche durch die Züchtung des erzeugenden Stammes von Candida guilliermondii unter aeroben Bedingungen auf einem eine durch den Pilz assimilierbare Kohlenstoff- und Stickstoffquelle enthaltenden Nährboden bei 24 bis 42 °C, vorzugsweise während 48 bis 72 Stunden, erhalten worden ist, ausgegangen, da dann bei der Hauptgärung die Vermehrung des Stammes wesentlich besser ist.

Für die Züchtung eines Stammes von Candida guilliermondii unter aeroben Bedingungen bei 24 bis 42 °C durch Züchtung auf einem eine durch den Pilz assimilierbare Kohlenstoff- und Stickstoffquelle enthaltenden Nährboden zur Herstellung der Vorkultur sowie die weitere Züchtung [einschließlich der Verlängerung der Züchtung beziehungsweise der Weitervermehrung bei den Varianten a) und b) des erfindungsgemäßen Verfahrens] können als assimilierbare Kohlenstoffquelle vorteilhaft folgende Stoffe eingesetzt werden : Glucose, Rohrzucker (Saccharose), Maltose, Cellobiose, Melibiose, Melecitose, Inulin, Arbutin, Galaktose, Sorbose, Trehalose, Ribose, Xylose, L-Arabinose, D-Arabinose, Glycerin, Ribit, D-Mannit, D-Dulcit, L-Methyl-D-glykoside, Salicin, Bernsteinsäure und Citronensäure. Die für die Züchtung eines Stammes von Candida guilliermondii unter aeroben Bedingungen bei 24 bis 42 °C durch Züchtung auf einem eine durch den Pilz assimilierbare Kohlenstoff- und Stickstoffquelle enthaltendenden Nährboden zur Herstellung der Vorkultur sowie die weitere Züchtung verwendeten Nährböden können als assimilierbare Stickstoffquelle vorteilhaft die folgenden Stoffe enthalten : Serum, « Proteose peptone » (Difco certified, Difco Laboratories, Detroit, Michigan, USA) und andere Proteosen, Peptone und Aminosäuren enthaltende Präparate.

Bei der Herstellung der immunbiologischen Präparate Cg1 und Cg2 nach dem erfindungsgemäßen Verfahren kann zweckmäßig wie folgt vorgegangen werden. Am Ende des Züchtungsabschnittes wird die Bakterienfreiheit kontrolliert, dann werden die Pilzzellen zweckmäßig durch Zentrifugieren von der Fermentbrühe isoliert. Die isolierten Zellen werden mit Wasser oder einer wäßrigen Salzlösung, vorzugsweise einer physiologischen Natriumchloridlösung, gewaschen. Die isolierten Zellen von Candida guilliermondii werden dann in an sich bekannter Weise mechanisch aufgeschlossen. Es kann auch so vorgegangen werden, daß der Niederschlag getrocknet und dann in einem sterilen Mahlwerk gemahlen wird. Zwischendurch wird vom Mahlgut von Zeit zu Zeit eine Probe genommen und der Grad des Aufschlusses mikroskopisch kontrolliert. Danach werden die mechanisch aufgeschlossenen Zellen vorzugsweise mit einer physiologischen Kochsalzlösung extrahiert. Nach einer anderen Verfahrensweise werden die isolierten Zellen von Candida guilliermondii mit Wasser oder einer wäßrigen Salzlösung, vorzugsweise einer physiologischen Kochsalzlösung, vermischt und die Zellsuspension mit Ultraschall behandelt. Von der Suspension wird von Zeit zu Zeit eine Probe entnommen und der Fortschritt des Aufschlusses mikroskopisch kontrolliert. Bei beiden Behandlungsverfahrensweisen erfolgt gleichzeitig mit oder nach der mechanischen Zerstörung die Extraktion, vorteilhaft mit einer wäßrigen Lösung, vorzugsweise einer physiologischen Kochsalzlösung. Dann wird der Feststoff (Zellenbruchstücke) durch Zentrifugieren entfernt, die überstehende Flüssigkeit filtriert und dem Filtrat ein Alkohol zugesetzt. Der erhaltene Niederschlag kann gegebenenfalls durch zur Reinigung der Eiweisse verwendete allgemein bekannte Verfahrensweisen, zum Beispiel Chromatographieren, Ultrafiltrieren, Dialyse oder Elektrophorese, gereinigt werden ; wenn das immunbiologische Präparat jedoch für diagnostische Zwecke verwendet werden soll, ist eine weitere Reinigung des Niederschlages nicht unbedingt notwendig. Es wurde bereits erwähnt, daß beim immunbiologischen Präparat Cg2 für diagnostische Zwecke der Auszug der aufgeschlossenen Zellen direkt verwendbar ist. Die Bezeichnung « immunbiologisches Präparat » schließt auch diesen für diagnostische Zwecke direkt geeigneten Auszug ein. In den weiteren Fällen wird der Niederschlag in an sich bekannter Weise unter Verwendung von üblichen Trägerstoffen, zum Beispiel von physiologischer Kochsalzlösung, durch übliche verfahrenstechnische beziehungsweise technologische Vorgänge, zum Beispiel Lösen, Einfrieren oder Lyophilisieren, zu einem immunbiologischen Präparat umgesetzt.

Bei der Herstellung der immunbiologischen Präparate Cg3 nach dem erfindungsgemäßen Verfahren kann zweckmäßig wie folgt vorgegangen werden. Die Zellen von Candida guilliermondii werden am Ende des Züchtungsabschnittes unter Verwendung von Zellgiften, zum Beispiel Formaldehyd, in der Kultur abgetötet. Es kann auch der Kultur eine 0,5 %ige wäßrige Formaldehydlösung zugesetzt werden und sie dann 6 Stunden lang bei 37 °C bebrütet werden. Die abgetöteten Zellen können durch Waschen gereinigt werden. Als Waschflüssigkeit kann vorteilhaft eine physiologische Kochsalzlösung eingesetzt werden. Dann werden die abgetöteten Zellen durch dür die Herstellung von Diagnostica im Laboratorium geeignete an sich bekannte Verfahren, zum Beispiel Suspendieren in einem wäßrigen Medium oder Einfrieren, zu immunbiologischen Präparaten umgesetzt.

Die erfindungsgemäß hergestellten, zum Beispiel in Form von Kristallampullen vorliegenden, immunbiologischen Präparate Cg1, Cg2 und Cg3 können zur Feststellung von Infektionen durch Candida guilliermondii an lebenden Organismen zweckmäßig wie folgt eingesetzt werden.

Die Präparate Cg1 und Cg2 können für Untersuchungen sowohl unter Bedingungen in vitro als auch unter Bedingungen in vivo eingesetzt werden, während das Präparat Cg3 nur für Untersuchungen unter Bedingungen in vitro anwendbar ist. Zweckmäßig wird wie folgt vorgegangen.

Bei den Untersuchungen unter Bedingungen in vivo wird dem zu untersuchenden Individuum intracutan ein Präparat Cg1 oder Cg2 in Form einer Injektion verabreicht. Das Auftreten der Infektion

14

durch Candida guilliermondii wird mit der durch das Präparat Cg1 ausgelösten allergischen Frühreaktion 1, 6 und 12 Stunden nach der Verabreichung der intracutanen Injektion beurteilt : Bei den infizierten Individuen wird an der Impfungsstelle eine wachsende, gut unterscheidbare Schwellung sichtbar, die 24 Stunden nach der Impfung nicht mehr zu beobachten ist. Das Auftreten einer Infektion durch Candida guilliermondii kann mit Hilfe der durch das Präparat Cg2 ausgelösten, wie bei einer Tuberkulin-Impfung auftretenden allergischen Spätreaktion 36 bis 48 Stunden nach der intracutanen Injektion beurteilt werden.

Die immunbiologischen Präparate Cg1, Cg2 und Cg3 können für die unter Bedingungen *in vitro* durchgeführten diagnostischen (Laboratoriums)-Untersuchungen zweckmäßig wie folgt angewandt werden.

Die Grundlage jedes Nachweises ist immer die immunbiologische Niederschlagsbildungsreaktion, bei der negatives Serum, Serum mit hohem Agglutiningehalt sowie solches mit hohem Präzipitingehalt als Reagenzien eingesetzt werden. Der Gegenstand der Erfindung umfaßt wie bereits erwähnt auch die Herstellung von Serum mit hohem Agglutiningehalt und von Serum mit hohem Präzipitingehalt.

Unter « negativem Serum » ist das Serum von Rindern oder anderen Versuchstieren, die bei der mit den erfindungsgemäß hergestellten immunbiologischen Präparaten Cg1, Cg2 beziehungsweise Cg3 durchgeführten Untersuchungen negativ reagieren, zu verstehen.

Das Serum mit hohem Agglutiningehalt kann wie bereits erwähnt in der Weise hergestellt werden, daß Kaninchen mit dem erfindungsgemäß hergestellten immunbiologischen Präparat Cg2 oder Cg3 auf übliche Weise immunisiert werden, dann die Tiere entblutet werden und das Serum der Tiere auf übliche Weise isoliert wird.

Das Serum mit hohem Präzipitingehalt kann wie bereits erwähnt in der Weise hergestellt werden, daß Kaninchen mit dem immunbiologischen Präparat Cg1 auf übliche Weise immunisiert werden, dann die Tiere entblutet werden und das Serum der Tiere auf bekannte Weise isoliert wird. Serum mit hohem Präzipitingehalt kann auch in der Weise erhalten werden, daß das Serum von unter natürlichen Bedingungen erkrankten Rindern zum Beispiel durch die Fällungsverfahrensweise [Präzipitationsverfahrensweise], für die ein Durham- oder Kapillarröhrchen verwendet wird, mit Cg1-Allergen untersucht wird, die Tiere mit Serum, das einen hohen Präzipitin-Titerwert hat, entblutet werden und dann das Serum auf bekannte Weise isoliert wird.

Im Besitz der obigen Reagenzien sowie der immunbiologischen Präparate Cg1, Cg2 und Cg3 können folgende Laboratoriumsuntersuchungen durchgeführt werden :

I. Mit einem Durham- oder Kapillarröhrchen durchgeführte Fällungsprobe [Präzipitationsprobe] mit dem immunbiologischen Präparat Cg1 :

In je 1 Durhamröhrchen wird negatives Serum beziehungsweise Serum mit hohem Präzipitingehalt mit einer Pasteurpipette so eingewogen, daß sich auf der Oberfläche des Serums keine Luftblasen befinden. Etwa die Hälft jedes Durhamröhrchens wird mit Serum gefüllt. 2 Durhamröhrchen werden auf die gleiche Weise mit dem zu untersuchenden Serum gefüllt. Das immunbiologische Präparat Cg1 wird in Form einer Lösung eingesetzt, die mit einer physiologischen Kochsalzlösung hergestellt wurde. Die Lösung wird mit der Pasteurpipette so vorsichtig auf das zu untersuchende Serum im einen Durhamröhrchen sowie die Reagenzien (auf das negative Serum und das Serum mit hohem Präzipitingehalt) wie eine Schicht aufgetragen, daß sich die Flüssigkeiten nicht vermischen, sondern 2 Schichten bilden. Auf das zu untersuchende Serum im anderen Durhamröhrchen wird nur eine Schicht der physiologischen Kochsalzlösung als solcher aufgetragen. Die Röhrchen werden auf Raumtemperatur gehalten, nach 10 bis 15 Minuten beziehungsweise 1 Stunde werden die Beobachtungen aufgezeichnet, dann werden die Röhrchen 24 Stunden lang auf + 5 °C gehalten und daraufhin werden wieder die Beobachtungen aufgezeichnet. Wenn sich im zu untersuchenden Serum des ersten Durhamröhrchens Antikörper gegen das Antigen Candida guilliermondii (das Präparat Cg1) befinden, dann ist an der Grenzflächen der 2 Schichten ein Fällungsring [präzipitationsring] zu sehen.

II. Das immunbiologische Präparat Cg2 ist als Antigen bei Immundiffusions-, Latexagglutinations- und Gegenimmunelektrophoreseverfahren [Counter-Immunelektrophorese-Verfahren] einsetzbar.

Diese Verfahren werden auf die aus dem Fachschrifttum bekannte Weise durchgeführt (siehe zum Beispiel Palmer D. F. und Mitarbeiter : Serodiagnosis of mycotic, diseases, Charles C. Thomas, Illinois, USA [1977]).

III. Das immunbiologische Präparat Cg3 kann als Antigen in Agglutinations- und indirekten Fluoreszenzverfahren nach bekannten Verfahrensweisen (siehe zum Beispiel Palmer D. F. und Mitarbeiter : Serodiagnosis of mycotic diseases, Charles C. Thomas, Illinois, USA [1977]) oder zur Herstellung von gefärbten Antigen verwendet werden, das für die ABR-Probe (Abortus-Bang-Rinsprobe) notwending ist (Hutyra F., I. Marek, R. Manninger und I. Mocsy : Spezielle Pathologie und Therapie der Haustiere, VEB Gustav-Fischer-Verlag, Jena, 1959).

Das immunbiologische Präparat Cg2 kann auch zu vorbeugenden Immunisierung von Menschen und Tieren, die der Gefahr einer Infektion durch Candida guilliermondii ausgesetzt sind, eingesetzt werden. Für diesen Zweck wird das immunbiologische Präparat Cg2 in der bekannten Form von Impfstoffen beziehungsweise Vakzinen bereitgestellt. Die Impfstoffe beziehungsweise Vakzinen können gegebenenfalls außer dem Wirkstoff und dem Verdünnungsmittel auch einem Trägerstoff, zum Beispiel ein Aluminiumhydroxydgel, der das langsame stufenweise Absorbieren des Wirkstoffes sicherstellt, enthalten. Der Impfstoff kann durch intrakutane, intramuskuläre oder subkutane Injektion in den zu behandelnden Organismus eingebracht werden. Es wird vorteilhaft in der Weise vorgegangen, daß bei der Immunisierung 2 Wochen nach der ersten Impfung die behandelten Individuen erneut geimpft werden, wobei bei der zweiten Impfung mehr Antigen in den Organismus eingebracht wird als bei den ersten. Die bei der Immunisierung angewandte Dosis wird in Abhängigkeit von verschiedenen Faktoren, wie vom Alter und vom allgemeinen Gesundheitszustand des zu behandelnden Individuums, von der Art und vom Alter des Tieres, von vorhergehenden Immunisierungsbehandlungen und vom Grad der Gefährdetheit, variiert.

Das immunbiologische Präparat Cg1 kann auch zur Behandlung von Menschen und Tieren, die an einer Infektion durch Candida guilliermondii erkrankt sind, eingesetzt werden. In diesem Fall wird der zu behandelnde Mensch beziehungsweise das zu behandelnde Tier zuerst mit dem immunbiologischen Präparat Cg1 desensibilisiert und dann wird das immunbiologische Präparat Cg1 in Form einer intrakutanen, intramuskulären oder subkutanen Injektion in den Organismus eingebracht. Auch dieses immunbiologische Präparat Cg1 kann einen Trägerstoff, zum Beispiel Aluminiumhydroxydgel, der das langsame stufenweise Absorbieren des Wirkstoffes sicherstellt, enthalten. Bei der immunbiologischen Behandlung werden die Individuen 2-wöchentlich nach der ersten Impfung erneut geimpft, wobei mit fortschreitender Zahl der Impfungen die Menge des in den Organismus eingebrachten Antigens stufenweise gesteigert wird. Die für die Behandlung notwendige Dosis hängt von zahlreichen Faktoren, wie vom Alter des zu behandelnden Menschen beziehungsweise Tieres, vom Ausmaß und von der Schwere der Infektion, von der Tierart und von der Art und der Häufigkeit der Behandlung, ab.

Die Erfindung wird an Hand der folgenden Beispiele näher erläutert.

Beispiel 1

Herstellung eines immunbiologischen Präparates Cg1

Es wurden in einen 500 cm³ Schüttelkolben 250 cm³ eines flüssigen Standard-Nährbodens, der eine Dextrose/Pepton/Hefe-Suppe enthielt (siehe Palmer D. F. und Mitarbeiter : Serodiagnosis of mycotic diseases, Seite 49, Charles C. Thomas, Illinois, USA [1977]), gefüllt. Der Nährboden wurde mit einer aufrechterhaltenen Kultur von Candida guilliermondii Nr. CBS-566 beimpft und dann 48 Stunden lang bei 37 °C bebrütet. Während der Züchtung wurde der Kolben mit einer Geschwindigkeit von 130 Umdrehungen/Minute geschüttelt. Die so erhaltene Kultur wurde als Impfkultur für die nächste Stufe verwendet.

In eine 20 l Laboratoriumsgärvorrichtung wurden 10 l des obigen flüssigen Standard-Nährbodens gefüllt und dann wurde dem Nährboden die obige Impfkultur zugesetzt. Die Züchtung wurde unter Belüften und Rühren bei 37 °C durchgeführt. Die Vermehrung nahm nach 72 Stunden ab und hörte schließlich ganz auf. Die Züchtung konnte unter diesen Bedingungen noch 15 Tage fortgesetzt werden (aufrechterhaltene oder verlängerte Züchtung). Dann wurde der Kultur zur Gram-Färbung eine Probe entnommen. Wenn sich die Probe verfärbte, dann zeugte dies von Bakterienverunreinigungen, so daß die Kultur nicht mehr zur Herstellung von immunbiologischen Präparaten verwendet werden konnte. Wenn die Untersuchung zeigte, daß die Kultur keine Bakterienverunreinigung enthielt, wurde der Inhalt der Gärvorrichtung in eine Zentrifuge überführt und die Zellenstoffe des Pilzes wurden durch Zentrifugieren (10 Minuten lang mit einer Geschwindigkeit von 1 000 Umdrehungen/Minute) von der Flüssigkeitsphase isoliert. Der erhaltene Zellenstoff wurde 3-mal mit physiologischer Kochsalzlösung gewaschen, zentrifugiert (10 Minuten ; 1 000 Umdrehungen/Minute) und dann in einer physiologischen Kochsalzlösung suspendiert. Die Suspension wurde in einem X-Press-Gerät unter einem Druck von etwa 10 000 kg/cm² aufgeschlossen. Der Feststoff wurde durch Zentrifugieren (30 Minuten ; 3 000 Umdrehungen/Minute) entfernt und die überstehende Flüssigkeit wurde durch einen Seitz-Filtereinsatz abfiltriert und dem Filtrat wurden 4 Vol.-Teile Alkohol zugesetzt. Der abgeschiedene Niederschlag wurde abfiltriert und dann in einer physiologischen Kochsalzlösung gelöst und die Lösung wurde bei — 20 °C getrocknet. Die je nach der Gärung und dem Aufschluß erhaltenen etwa 500 mg des lyophilisierten pulverförmigen Produktes wurden in Form einer Pulverampulle fertiggestellt und als Allergen für die intrakutane Probe, die auf der allergischen Frühreaktion beruht, eingesetzt.

Beispiel 2

Herstellung eines immunbiologischen Präparates Cg2

Es wurden in einen 500 cm³ Schüttelkolben 250 cm³ eines flüssigen Standard-Nährbodens, der eine Dextrose/Pepton/Hefe-Suppe enthielt (siehe Palmer D. F. und Mitarbeiter : Serodiagnosis of mycotic diseases, Seite 49, Charles C. Thomas, Illinois, USA [1977]), gefüllt. Der Nährboden wurde mit einer aufrechterhaltenen Kultur von Candida guilliermondii CBS-566 beimpft und dann 48 Stunden lang bei 37 °C bebrütet. Während der Züchtung wurde der Kolben mit einer Geschwindigkeit von 130 Umdrehungen/Minute geschüttelt. Die so erhaltene Kultur wurde als Impfkultur für die nächste Stufe verwendet.

In eine 20 l Laboratoriumsgärvorrichtung wurden 10 l des obigen flüssigen Dextrose/Peton/Hefe-Suppe-Nährbodens gefüllt und dem Nährboden wurde die obige Impfkultur zugesetzt. Die Züchtung wurde unter Lüften und Rühren 72 Stunden lang bei 37 °C vorgenommen. Dann wurde der Kultur so viel von einer 30 bis 40 %-igen wäßrigen Formaldehydlösung zugesetzt, daß die endgültige Formaldehydkonzentration der Kultur 0,5 % betrug, was aus Sicherheitsgründen getan wurde. Dann wurde die Kultur unter Rühren noch 6 Stunden bei 37 °C bebrütet. Danach wurde der Kultur eine Probe entnommen und die Lebensfähigkeit des Pilzes durch Abimpfung auf Sabouraud-Dextrose-Agar kontrolliert (die Platten wurden 48 Stunden lang bei 37 °C bebrütet). Der Kultur wurde für die Gram-Färbung eine Probe entnommen. Wenn sich die Probe verfärbte, waren Bakterienverunreinigungen vorhanden ; in diesem Fall konnte die Kultur nicht mehr zur Herstellung von immunbiologischen Präparaten verwendet werden. Wenn die Untersuchung zeigte, daß die Kultur keine Bakterienverunreinigungen enthielt, wurde der Inhalt der Gärvorrichtung in eine Zentrifuge überführt und 10 Minuten lang mit einer Geschwindigkeit von 1 000 Umdrehungen/Minute zentrifugiert. Der Feststoff wurde isoliert, mit einer 0,85 %-igen wäßrigen Natriumchloridlösung gewaschen und zentrifugiert (10 Minuten lang mit einer Geschwindigkeit von 1 000 Umdrehungen/Minute). Dem Feststoff wurde eine 0,85 %-ige wäßrige Natriumchloridlösung zugesetzt und die erhaltene Zellensuspension wurde in einem X-Press-Gerät unter einem Druck von etwa 10 000 kg/cm$^2$ aufgeschlossen. Das erhaltene Zellenhomogenisat wurde 30 Minuten lang mit einer Geschwindigkeit von 5 000 Umdrehungen/Minute zentrifugiert und die überstehende Flüssigkeit wurde isoliert und durch einen Seitz-Filtereinsatz abfiltriert und schließlich wurden dem Filtrat 4 Vol.-Teile Alkohol zugesetzt. Der abgeschiedene Niederschlag wurde abfiltriert und dann in einer physiologischen Kochsalzlösung gelöst und die Lösung wurde bei — 20 °C getrocknet. Die je nach der Gärung und dem Aufschluß gewonnenen etwa 1 500 mg des lyophilisierten pulverförmigen Präparates konnten als Antigen nach der Titration für Latexagglutinations-, Immundiffusions- und Gegenimmunelektrophorese-Untersuchungen [Counter-Immunelektrophorese-Untersuchungen] sowie ferner für Untersuchungen in vivo, die auf später Überempfindlichkeit beruhen, eingesetzt werden.


Beispiel 3


Herstellung eines immunbiologischen Präparates Cg3

Es wurde auf einen schrägen Sabouraud-Dextrose-Agar eine aufrechterhaltene Kultur von Candida guilliermondii Nr. CBS-566 geimpft und dann 48 Stunden lang bei 37 °C bebrütet. Der auf der erhaltenen schrägen Agar-Kultur entwickelte Pilz wurde in einer Dextrose/Pepton/Hefe-Suppe, beispielsweise in der in den Beispielen 1 und 2 verwendeten, suspendiert und die Suspension wurde als Impfkultur für die nächste Stufe verwendet.

In einen 500 cm$^3$ Erlenmeyer-Kolben wurden 250 cm$^3$ des obigen flüssigen Dextrose/Pepton/Hefe-Suppe-Nährbodens gefüllt und dem Nährboden wurde die obige Impfkultur zugesetzt. Das erhaltene Gemisch wurde 48 Stunden lang bei 37 °C bebrütet. Während der Züchtung wurde der Kolben mit einer Geschwindigkeit von 130 Umdrehungen/Minute geschüttelt. Dann wurde der Kultur so viel von einer 30 bis 40 %-igen wäßrigen Formaldehydlösung zugesetzt, daß die endgültige Formaldehydkonzentration der Kultur 0,5 % betrug. Die Kultur wurde in die Schüttelmaschine zurückgebracht und 6 Stunden lang bei 37 °C mit einer Geschwindigkeit von 130 Umdrehungen/Minute geschüttelt. Dann wurde der Kultur eine Probe entnommen und die Lebensfähigkeit des Pilzes durch Abimpfung auf einen Sabouraud-Dextrose-Agar kontrolliert (die Platten wurden 48 Stunden lang bei 37 °C bebrütet). Der Kultur wurde für die Gram-Färbung eine Probe entnommen. Wenn sich die Probe verfärbte, waren Bakterienverunreinigungen vorhanden, das heißt, daß die Kultur für die Herstellung von immunbiologischen Präparaten nicht mehr verwendet werden konnte. Wenn die Untersuchung zeigte, daß keine Bakterienverunreinigungen vorhanden waren, wurde der Inhalt des Kolbens 10 Minuten lang mit einer Geschwindigkeit von 1 000 Umdrehungen/Minute zentrifugiert. Die als Feststoff erhaltenen ganzen Zellen wurden mit einer sterilen physiologischen Kochsalzlösung 3-mal gewaschen und zentrifugiert (10 Minuten lang mit einer Geschwindigkeit von 1 000 Umdrehungen/Minute), wodurch etwa 2,5 g feuchte Zellmasse erhalten wurden.

1 Vol.-Teil der so erhaltenen feuchten Zellmasse wurden 3 Vol.-Teile einer sterilen physiologischen Kochsalzlösung zugesetzt. Der Lösung wurde so viel von einer handelsüblichen Natrium-äthylmercurithiosalicylatlösung (Merthiolatlösung) zugesetzt, daß die endgültige Konzentration 1/10 000 betrug, und die erhaltene Zellsuspension wurde bei + 4 °C gelagert. Es wurde ein immunbiologisches Präparat, das in der Laboratoriumsdiagnostik für Röhrchenagglutinations- und indirekte Fluoreszenzuntersuchungen oder zur Herstellung von gefärbten Antigenen, die für die ABR-Probe (Abortus-Bang-Ringprobe) notwendig sind (Hutyra F., I. Marek, R. Manninger und I. Mocsy : Spezielle Pathologie und Therapie der Haustiere. VEB Gustav-Fischer-Verlag, Jena, 1959), erhalten.

# 0 082 890

**Patentansprüche**

1. Verfahren zur Herstellung von immunbiologischen Präparaten, geeignet für den Nachweis von Infektionen durch Candida guilliermondii, die Vorbeugung gegen Infektionen durch Candida guilliermondii und/oder die Behandlung von Infektionen durch Candida guilliermondii, durch Züchtung bis zum Erreichen des Endes der Vermehrungsfähigkeit erfolgendes Vermehren eines Stammes von Candida guilliermondii unter aeroben Bedingungen bei 24 bis 42 °C mittels Züchtung auf einem eine durch den Pilz assimilierbare Kohlenstoff- und Stickstoffquelle enthaltenden Nährboden, dadurch gekennzeichnet, daß man die beim Vermehren erhaltene(n) Bevölkerung(en) für die verlängerte Züchtung unter identischen Bedingungen hält, die Pilzzellen von der Kultur trennt und wäscht oder trocknet sowie in an sich bekannter Weise auf mechanischem Wege aufschließt und extrahiert, den Auszug mit einem polaren organischen Lösungsmittel behandelt und den erhaltenen Niederschlag, gegebenenfalls nach weiterer Reinigung, in an sich bekannter Weise zu einem für immunbiologische Zwecke geeigneten Präparat formuliert.

2. Verfahren zur Herstellung von immunbiologischen Präparaten, geeignet für den Nachweis von Infektionen durch Candida guilliermondii, die Vorbeugung gegen Infektionen durch Candida guilliermondii und/oder die Behandlung von Infektion durch Candida guilliermondii, durch 48 bis 72 Stunden langes Vermehren eines Stammes von Candida guilliermondii unter aeroben Bedingungen bei 24 bis 42 °C mittels Züchtung auf einem eine durch den Pilz assimilierbare Kohlenstoff- und Stickstoffquelle enthaltenden Nährboden, dadurch gekennzeichnet, daß man nach gegebenenfalls erfolgendem Weitervermehren der beim Vermehren erhaltene(n) Bevölkerung(n), vorzugsweise bis zur Herstellung von 2 bis 3 neuen Bevölkerungen, die Pilzzellen von der Kultur trennt und wäscht oder trocknet sowie in an sich bekannter Weise auf mechanischem Wege aufschließt und extrahiert sowie gegebenenfalls zum Auszug ein polares organisches Lösungsmittel zugibt und den erhaltenen Niederschlag, eventuell nach weiterer Reinigung, in an sich bekannter Weise zu einem für immunbiologische Zwecke geeigneten Präparat formuliert.

3. Verfahren zur Herstellung von immunbiologischen Präparaten, geeignet für den Nachweis von Infektionen durch Candida guilliermondii, die Vorbeugung gegen Infektion durch Candida guilliermondii und/oder die Behandlung von Infektionen durch Candida guilliermondii, durch 48 bis 72 Stunden langes Vermehren eines Stammes von Candida guilliermondii unter aeroben Bedingungen bei 24 bis 42 °C mittels Züchtung auf einem eine durch den Pilz assimilierbare Kohlenstoff- und Stickstoffquelle enthaltenden Nährboden, dadurch gekennzeichnet, daß man die beim Vermehren erhaltene Kultur abtötet, die abgetöteten Zellen abtrennt und, gegebenenfalls nach weiterer Reinigung, in an sich bekannter Weise zu einem für immunbiologische Zwecke geeigneten Präparat formuliert.

4. Verfahren nach Anspruch 1 oder 2, dadurch gekennzeichnet, daß man bei der Behandlung des Auszuges als polares organisches Lösungsmittel 1 oder mehr Alkanol(e) mit 1 bis 4 Kohlenstoffatom(en) verwendet.

5. Verfahren nach Anspruch 1, 2 oder 4, dadurch gekennzeichnet, daß man als polares organisches Lösungsmittel Äthanol verwendet.

6. Verfahren nach Anspruch 1 bis 5, dadurch gekennzeichnet, daß man das Formen beziehungsweise Umsetzen zum für immunbiologische Zwecke geeigneten Präparat zu einer Form, welche in der Human- oder Tiermedizin anwendbar ist, durchführt.

7. Verfahren nach Anspruch 1, 2 oder 4 bis 6, dadurch gekennzeichnet, daß man das Formen zum für immunbiologische Zwecke geeigneten Präparat zu einem Allergen, löslichen Antigen oder Impfstoff durchführt.

8. Verfahren nach Anspruch 3 oder 6, dadurch gekennzeichnet, daß man das Formen zum für immunbiologische Zwecke geeigneten Präparat zu einem korpuskulären Antigen durchführt.

9. Verfahren nach Anspruch 1 bis 8, dadurch gekennzeichnet, daß man beim Vermehren beziehungsweise Weitervermehren Temperaturen von 32 bis 38 °C anwendet.

10. Verfahren nach Anspruch 1, 2, 4 bis 7 oder 9, dadurch gekennzeichnet, daß man zum Waschen und/oder Extrahieren eine physiologische Kochsalzlösung verwendet.

11. Verfahren nach Anspruch 1, 4 bis 7, 9 oder 10 zur Herstellung von Serum mit hohem Präzipitingehalt, geeignet als Reagens für den Laboratoriumsnachweise von Infektionen durch Candida guilliermondii, dadurch gekennzeichnet, daß man Kaninchen mit nach Anspruch 1, 4 bis 7, 9 oder 10 hergestellten immunbiologischen Präparaten auf übliche Weise immunisiert, dann die Tiere entblutet und das Serum der Tiere auf übliche Weise isoliert.

12. Verfahren nach Anspruch 2 bis 10 zur Herstellung von Serum mit hohem Agglutiningehalt, geeignet als Reagens für den Laboratoriumsnachweis von Infektionen durch Candida guilliermondii, dadurch gekennzeichnet, daß man Kaninchen mit nach Anspruch 2 bis 10 hergestellten immunbiologischen Präparaten auf übliche Weise immunisiert, dann die Tiere entblutet und das Serum der Tiere auf übliche Weise isoliert.

**Claims**

1. A process for preparing immuno-biological compositions suitable for the detection of infections

18

by Candida guilliermondii, the prophylaxis against infections by Candida guilliermondii and/or the treatment of infections by Candida guilliermondii by proliferation until the proliferation capability has been reached of a strain of Candida guilliermondii under aerobic conditions at 24 to 42 °C by cultivation on a nutritive medium containing a carbon source and a nitrogen source being assimilable by the fungus, characterized in that the population(s) obtained by the proliferation is (are) kept for a prolongated cultivation under identical conditions, the fungus cells are separated from the culture, are washed or dried and in a manner known *per se* disrupted mechanically and extracted, the extract is treated with a polar organic solvent and the obtained precipitate, optionally after an additional purification, in a manner known *per se* is formulated to a composition suitable for immuno-biological purposes.

2. A process for preparing immuno-biological compositions suitable for the detection of infections by Candida guilliermondii, the prophylaxis against infections by Candida guilliermondii and/or the treatment of infections by Candida guilliermondii by 48 to 72 hours lasting proliferation of a strain of Candida guilliermondii under aerobic conditions at 24 to 42 °C by cultivation on a nutritive medium containing a carbon source and a nitrogen source being assimilable by the fungus, characterized in that after an optionally occurred additional proliferation of the population(s) obtained by the proliferation, preferably up to the preparation of 2 to 3 new populations, the fungus cells are separated from the culture, are washed and dried and are disrupted mechanically in a manner known *per se,* and extracted, optionally a polar organic solvent is added to the extract and the obtained precipitate, optionally after an additional purification, in a manner known *per se* is formulated to a composition suitable for immuno-biological purposes.

3. A process for preparing immuno-biological compositions suitable for the detection of infections by Candida guilliermondii, the prophylaxis against infections by Candida guilliermondii and/or the treatment of infections by Candida guilliermondii by 48 to 72 hours lasting proliferation of a strain of Candida guilliermondii under aerobic conditions at 24 to 42 °C by cultivation on a nutritive medium containing a carbon source and a nitrogen source being assimilable by the fungus, characterized in that the culture obtained by proliferation is killed, the killed cells are separated, and optionally after an additional purification are formulated in a manner known *per se* to a composition suitable for immuno-biological purposes.

4. A process according to claim 1 or 2, characterized in that as polar organic solvent for the treatment of the extract 1 ore more alkanol(s) having 1 to 4 carbon atom(s) is (are) unsed.

5. A process according to claim 1, 2 or 4, characterized in that as polar organic solvent ethanol is used.

6. A process according to claim 1 to 5, characterized in that the formulation and convertion, respectively to a composition suitable for immuno-biological purposes is carried out to a form being suitable in the human medical science or veterinary science.

7. A process according to claim 1, 2 or 4 to 6, characterized in that the formulation to a composition suitable for immuno-biological purposes is carried out to obtain a allergen, a soluble antigen or a vaccine.

8. A process according to claim 3 or 6, characterized in that the formulation to a composition suitable for immuno-biological purposes is carried out to obtain a corpuscular antigen.

9. A process according to claim 1 to 8, characterized in that for the proliferation and additional proliferation, respectively temperatures of 32 to 38 °C are used.

10. A process according to claims 1, 2, 4 to 7 or 9, characterized in that for the washing and/or the extraction a physiological salt solution is used.

11. A process according to claim 1, 4 to 7, 9 or 10 for preparing a serum having a high precipitine content being suitable as reagent for the laboratory detection of infections by Candida guilliermondii, characterized in that in a manner known *per se* rabbits are immunized with immuno-biological composition prepared according to claim 1, 4 to 7, 9 or 10, the blood is removed from the animals and the serum of the animals is separated in the usual manner.

12. A process according to claim 2 to 10 for the preparation of a serum having a high agglutinine content and being suitable as reagent for the laboratory detection of infections by Candida guilliermondii, characterized in that in a manner known *per se* rabbits are immunized with immuno-biological compositions according to claim 2 to 10, the blood is removed from the animals and the serum of the animals is separated in the usual manner.

**Revendications**

1. Procédé de préparation de produits immunologiques appropriés pour déceler des infections dues à Candida guilliermondii, pour prévenir des infections dues à Candida guilliermondii et/ou pour traiter des infections dues à Candida guilliermondii, par multiplication, obtenue par culture jusqu'à obtention de la limite des possibilités de multiplication, d'une souche de Candida guilliermondii dans des conditions aérobiques à une température de 24 à 42 °C par culture sur un milieu nutritif renfermant une source de carbone et d'azote assimilable par le champignon, caractérisé en ce qu'on maintient la ou les population(s) obtenue(s) lors de la multiplication, pour la culture prolongée, dans des conditions identiques, on sépare les cellules de champignons de la culture, et on les lave ou on les sèche, et on les dissout ensuite, de façon connue en soi, par des moyens mécaniques et on les extrait, on traite l'extrait par un solvant organique polaire et l'on met le précipité obtenu, éventuellement après purification ultérieure, de façon connue en soi, sous la forme d'un produit convenant pour des applications immunologiques.

2. Procédé de préparation de produits immunologiques appropriés pour déceler des infections dues à Candida guilliermondii, pour prévenir des infections dues à Candida guilliermondii et/ou pour traiter des infections dues à Candida guilliermondii, par multiplication d'une durée de 48 à 72 heures d'une souche de Candida guilliermondii dans des conditions aérobiques à une température de 24 à 42 °C par culture sur un milieu nutritif renfermant une source de carbone et d'azote assimilable par le champignon, caractérisé en ce qu'après avoir poursuivi éventuellement la multiplication de la population ou des populations obtenue(s) lors de la multiplication, de préférence jusqu'à obtention de 2 à 3 nouvelles populations, on sépare les cellules de champignons de la culture, et on les lave ou on les sèche, et on les dissout de façon connue en soi par des moyens mécaniques et on les extrait, et l'on ajoute éventuellement à l'extrait un solvant organique polaire, et l'on met le précipité obtenu, éventuellement après avoir poursuivi la purification, de façon connue en soi, sous la forme d'un produit convenant pour des applications immunologiques.

3. Procédé de préparation de produits immunologiques appropriés pour déceler des infections dues à Candida guilliermondii, pour prévenir des infections dues à Candida guilliermondii et/ou pour traiter des infections dues à Candida guilliermondii, par multiplication d'une durée de 48 à 72 heures d'une souche de Candida guilliermondii dans des conditions aérobiques à une température de 24 à 42 °C par culture sur un milieu nutritif renfermant une source de carbone et d'azote assimilable par le champignon, caractérisé en ce qu'on provoque l'extinction de la culture obtenue lors de la multiplication, on sépare les cellules mortes et, de façon connue en soi, éventuellement après purification ultérieure, on les met de façon connue en soi, sous la forme d'un produit convenant pour des applications immunologiques.

4. Procédé selon la revendication 1 ou la revendication 2, caractérisé en ce qu'on utilise, lors du traitement de l'extrait, comme solvant organique polaire, un ou plusieurs alcanol(s) comportant 1 à 4 atomes de carbone.

5. Procédé selon les revendications 1, 2 ou 4, caractérisé en ce qu'on utilise, comme solvant organique polaire, de l'éthanol.

6. Procédé selon l'une quelconque des revendications 1 à 5, caractérisé en ce qu'on effectue la formation ou la transformation en un produit convenant pour des applications immunologiques, en le mettant sous une forme applicable en médecine humaine ou vétérinaire.

7. Procédé selon l'une quelconque des revendications 1, 2 et 4 à 6, caractérisé en ce qu'on effectue la mise sous la forme d'un produit convenant pour des applications immunologiques en le mettant sous la forme d'un allergène, d'un antigène soluble ou d'un agent d'inoculation.

8. Procédé selon la revendication 3 ou la revendication 6, caractérisé en ce qu'on effectue la mise sous la forme d'un produit convenant pour des applications immunologiques en le mettant sous la forme d'un antigène corpusculaire.

9. Procédé selon l'une quelconque des revendications 1 à 8, caractérisé en ce qu'on applique, lors de la multiplication ou de la multiplication ultérieure, des températures de 32 à 38 °C.

10. Procédé selon l'une quelconque des revendications 1, 2, 4 à 7 et 9, caractérisé en ce qu'on utilise pour le lavage et/ou l'extraction une solution physiologique de chlorure de sodium.

11. Procédé selon l'une quelconque des revendications 1, 4 à 7, 9 et 10, pour préparer du sérum de forte teneur en précipitine, convenant comme réactif pour déceler au laboratoire des infections dues à Candida guilliermondii, caractérisé en ce qu'on immunise de façon usuelle des lapins avec des produits immunologiques préparés selon l'une quelconque des revendications 1, 4 à 7, 9 et 10, puis l'on extrait le sang des animaux et l'on isole le sérum des animaux de façon usuelle.

12. Procédé selon l'une quelconque des revendications 2 à 10, pour préparer du sérum de forte teneur en agglutinine, convenant comme réactif pour déceler au laboratoire des infections dues à Candida guilliermondii, caractérisé en ce qu'on immunise de façon usuelle des lapins avec des produits immunologiques préparés selon l'une quelconque des revendications 2 à 10, puis l'on extrait le sang des animaux et l'on isole le sérum des animaux de façon usuelle.